(19) Europäisches Patentamt
European Patent Office
Office européen des brevets

(11) EP 4 098 265 A1

(12) # EUROPEAN PATENT APPLICATION

published in accordance with Art. 153(4) EPC

(43) Date of publication:
**07.12.2022 Bulletin 2022/49**

(21) Application number: **20916420.1**

(22) Date of filing: **25.03.2020**

(51) International Patent Classification (IPC):
***A61K 31/7048*** $^{(2006.01)}$ ***A61K 45/06*** $^{(2006.01)}$
***A61P 31/14*** $^{(2006.01)}$ ***A61P 31/18*** $^{(2006.01)}$

(86) International application number:
**PCT/CN2020/081204**

(87) International publication number:
**WO 2021/151264 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.01.2020 CN 202010077555**
**10.02.2020 CN 202010085611**

(71) Applicant: **Shenyang Fuyang Pharmaceutical Technology Co., Ltd.**
**Shenyang, Liaoning 110164 (CN)**

(72) Inventors:
- **JIANG, Enhong**
  **Shenyang, Liaoning 110164 (CN)**
- **JIANG, Jiandong**
  **Shenyang, Liaoning 110164 (CN)**
- **CHE, Yongsheng**
  **Shenyang, Liaoning 110164 (CN)**
- **LI, Yuhuan**
  **Shenyang, Liaoning 110164 (CN)**
- **WANG, Dong**
  **Shenyang, Liaoning 110164 (CN)**
- **HE, Weiqing**
  **Shenyang, Liaoning 110164 (CN)**
- **ZHAO, Xiaofeng**
  **Shenyang, Liaoning 110164 (CN)**

(74) Representative: **ABG Intellectual Property Law, S.L.**
**Avenida de Burgos, 16D**
**Edificio Euromor**
**28036 Madrid (ES)**

(54) **USE OF ACYLATED SPIRAMYCIN IN PREPARATION OF MEDICAMENT FOR TREATING CORONAVIRUS INFECTIOUS DISEASE**

(57) Disclosed is an application of acylated spiramycin in the preparation of medicine for treating coronavirus diseases. Computer simulation is adopted to prove that isovalerylspiramycin I, one of the main active ingredients of carrimycin, binds to coronavirus S protein and its host cell receptor ACE2 protein; it shows that isovalerylspiramycin I has potential inhibitory activity on ACE2. The ACE2 expression is decreased after coronavirus infection, which in turn leads to the increase of lung injury. The combination of isovalerylspiramycin I and ACE2 can inhibit the function of ACE2 on the one hand, and increase the stability of ACE2 on the other hand, inhibit the decrease of ACE2 expression after coronavirus infection. Isovalerylspiramycin I can also strongly bind to the coronavirus main protease Mpro inhibition site, and inhibit the transcription and replication of coronaviruses. Both in vitro experiment and in vivo experiment show that carrimycin and its main active ingredients can inhibit coronaviruses and diseases caused by coronaviruses.

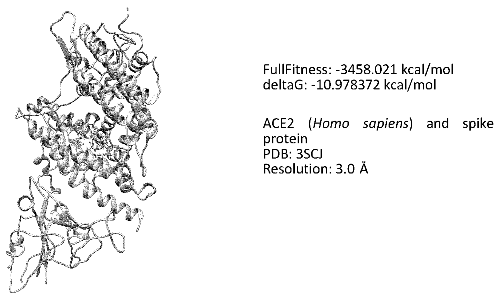


Fig. 1

EP 4 098 265 A1

## Description

### TECHNICAL FIELD

The present disclosure relates to new uses of antibiotics, especially macrolide antibiotics or isovalerylspiramycin compounds or combinations thereof, in particular to application of acylated isovalerylspiramycin analogues or a combination thereof in the preparation of medicine for treating coronavirus infection diseases.

### BACKGROUND

Isovalerylspiramycin analogues are a series of antibiotics produced from genetic engineering bacteria constructed by integrating a carbomycin 4"-isovaleryltransferase gene into the chromosome of Streptomyces spiramyceticus F21 using the homologous recombination technology by the research group of Professor WANG Yiguang from the Bioengineering Room of the Institute of Medicinal Biotechnology of Chinese Academy of Medical Sciences. As a major special project of the Ministry of Science and Technology, this hybrid antibiotic is a national first-in-class new drug with the common name of carrimycin. Carrimycin is a national first-in-class innovative drug jointly developed by the Institute of Medicinal Biotechnology of Chinese Academy of Medical Sciences and Shenyang Tonglian Group Co., Ltd.. Carrimycin is of a multi-component small-molecule structure, and is a chemical mixture composed of isovalerylspiramycin III, isovalerylspiramycin II and isovalerylspiramycin I serving as three main ingredients, and nine ingredients including a certain amount of butyrylspiramycin III, propionylspiramycin III, acetylspiramycin III, butyrylspiramycin II, propionylspiramycin II, acetylspiramycin II and the like.

isovalerylspiramycin I: molecular formula: C48H82N2O15 molecular weight: 926.00
isovalerylspiramycin II: molecular formula: C50H84N2O16 molecular weight: 968.00
isovalerylspiramycin III: molecular formula: C51H86N2O16 molecular weight: 982.00

The structure of the main ingredients of carrimycin is as follows:

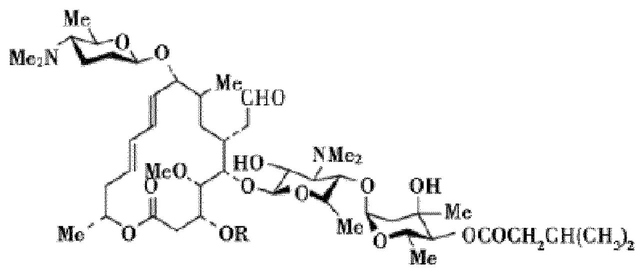

R= H, spiramycin I or 4"-isovalerylspiramycin I ;
R= $CH_3CO$, spiramycin II or 4"-isovalerylspiramycin II ;
R= $CH_3CH_2CO$, spiramycin III or 4"-isovalerylspiramycin III

I, II and III in the above formula are isovalerylspiramycin compounds I, II and III.

The action mechanism of spiramycin is that it binds specifically with the 50S subunit of a bacterial ribosome and interferes with the synthesis of bacterial proteins by preventing the nascent peptide chain elongation. The degradation products of spiramycin in vivo are the degradation products after demycarose, the activity of which decreases or disappears, but after 4" acylation, it turns into the degradation pathway of deforosamine, and forms the products such as Platamycin A1 which still has antibacterial activity. The 4" isovalerylated side chain can also improve its stability in vivo, delay hydrolysis and prolong its half-life pharmacokinetics. Studies have shown that when the 4" position of spiramycin is acylated, the antibacterial activity of spiramycin in vivo is enhanced, including some macrolide antibiotic-resistant bacteria.

Studies have shown that isovalerylspiramycin analogues have strong antibacterial activity and significant inhibitory activity against mycoplasma and chlamydia, and have no obvious cross-resistance to similar medicines. In addition to being effective against drug-resistant Gram-positive bacteria (such as methicillin-resistant Staphylococcus aureus and drug-resistant Streptococcus pyogenes), they also have good curative effect against some β-lactamase-producing bacteria, and have good activity against some Gram-negative bacteria (such as Clostridium difficile and bacillus influenzae) and fungal Candida albicans.

Because the isovalerylspiramycin analogues have the advantages of low toxicity, small dose, few taking times

and convenient taking, they are the first choice for patients to pursue safe and effective medicines within the scope of indications; they are also significant to develop its potential new uses.

A coronavirus virus is a positive single stranded RNA virus with envelope, with a diameter of about 80-120 nm. Its genetic material is the largest among all RNA viruses, and only infects human beings, mice, pigs, cats, dogs and poultry vertebrates.

In 1975, the National Virus Naming Committee officially named Coronaviridae. Up to now, about 15 different coronavirus strains have been found, which can infect a variety of mammals and birds, and some of them can make human beings sick.

From the end of 2019 to January 2020, unexplained pneumonia occurred in some areas. The bronchoalveolar lavage samples of patients were sent for a new generation of sequencing, and a novel coronavirus was found. The whole genome sequence was more than 85% consistent with the bat-derived coronavirus sequence that caused the SARS epidemic. The novel coronavirus (SARS-CoV-2) strain was isolated and cultured. After transfection into human airway epithelial cells, cytopathic changes were observed, and free virus particles were found outside the cell and inclusion bodies filled with virus particles were found inside the periplasm membrane sac, which further confirmed that the coronavirus was the seventh coronavirus family member that could infect human beings.

As of January 29, 2020, there were more than 6,000 confirmed cases, more than 9,000 suspected cases and more than 100 deaths of the COVID-19 in China.

In the early morning of January 25, NEJM published online an article about original research and opinions on etiological confirmation of unexplained pneumonia in Wuhan, pointing out that 2019-nCoV was the third coronavirus in humans in the past 20 years. The other two coronaviruses which can cause SARS and MERS broke out on a large scale, causing a high mortality rate among infected people. At present, no specific anti-coronavirus medicines or vaccines have been proved to be effective for human beings. These genome sequence studies will promote the development of antiviral medicines, vaccines and experimental animal models.

On February 12, 2020, WHO named the disease caused by the novel coronavirus (SARS-CoV-2) as Corona Virus Disease 2019, COVID-19. At present, the number of confirmed cases of 2019-nCoV pneumonia is still increasing, and the situation of epidemic prevention and control is still grim. Therefore, it is urgent to find effective therapeutic medicines, especially those already on the market, to treat COVID-19.

## SUMMARY

The first object of the present disclosure is to provide an application of acylated spiramycin compounds or compositions thereof in the preparation of medicine for treating coronavirus infection diseases, in particular, an application of isovalerylspiramycin compounds or compositions thereof in the preparation of medicine for treating coronavirus infection diseases, and specifically, an application of carrimycin in the preparation of medicine for treating coronavirus infection diseases.

The second object of the present disclosure is to provide an application of acylated spiramycin compounds and compositions thereof in the preparation of medicine for treating 2019-nCoV infection diseases, in particular, an application of isovalerylspiramycin compounds and compositions thereof in the preparation of medicine for treating 2019-nCoV infection diseases, and specifically, an application of carrimycin in the preparation of medicine for treating diseases caused by 2019-nCoV.

The third object of the present disclosure is to provide an application of acylated spiramycin compounds or compositions thereof in the preparation of medicine for treating complications caused by coronavirus infection, in particular, an application of isovalerylspiramycin compounds or compositions thereof in the preparation of medicine for treating complications caused by coronavirus infection diseases, specifically, an application of carrimycin in the preparation of medicine for treating complications caused by coronavirus diseases, and more specifically, an application of carrimycin in the preparation of medicine for treating complications caused by 2019-nCoV pneumonia.

In order to achieve the above objects, the technical solution adopted by the present disclosure is as follows:

An application of acylated spiramycin compounds or compositions thereof in the preparation of medicine for treating coronavirus infection diseases, in particular, an application of isovalerylspiramycin compounds or compositions thereof in the preparation of medicine for treating coronavirus infection diseases, and specifically, an application of carrimycin in the preparation of medicine for treating coronavirus infection diseases;

an application of acylated spiramycin compounds and compositions thereof in the preparation of medicine for treating coronavirus infection diseases, particularly, an application of isovalerylspiramycin compounds and compositions thereof in the preparation of medicine for treating 2019-nCoV diseases, and specifically, an application of carrimycin in the preparation of medicine for treating 2019-nCoV diseases;

an application of antibiotics, especially macrolide antibiotic compounds, in the preparation of medicine for treating coronavirus infection diseases and other diseases, especially 2019-nCoV infection diseases.

The acylated spiramycin compounds in the present disclosure are at least selected from one of three main ingredients, namely isovalerylspiramycin I, isovalerylspiramycin II and isovalerylspiramycin III, and one of nine ingredients including a certain amount of butyrylspiramycin III, propionylspiramycin III, acetylspiramycin III, butyrylspiramycin II, propionylspiramycin II, acetylspiramycin II and the like, which are obtained by preparing carrimycin by a genetic engineering method, or a combination thereof, preferably selected from isovalerylspiramycin compounds, and more preferably selected from one of isovalerylspiramycins I, II, III, structural analogues thereof or a combination of at least two of them, or carrimycin. Among them, carrimycin is a national first-in-class new drug. Of course, It includs the above-mentioned compounds or compositions thereof obtained by any chemical or biological methods, or the analogues of carrimycin.

The coronaviruses in the present disclosure comprise $\alpha$ and $\beta$ coronaviruses, preferably: HCoV-229E, HCoV-OC43, SARS-CoV, HCoV-NL63, HCoV-HKU1, MERS-CoV, and 2019-nCoV, preferably SARS-CoV, MERS-CoV and 2019-nCoV, among which SARS is severe acute respiratory syndrome, MERS is the Middle East respiratory syndrome, and 2019-nCoV (COVID-19) is a novel coronavirus infection.

The coronavirus infection diseases in the present disclosure comprise respiratory tract, digestive tract and nervous system diseases, including but not limited to cold, fever, frontal sinusitis, otitis media, pharyngitis, chronic bronchitis, pneumonia, pleural effusion, various respiratory syndromes, acute gastroenteritis, cardiopulmonary diseases, hypoimmunity, repeated infection, lung injury, organ failure and other diseases; especially various diseases and complications, including various infectious diseases, caused by SARS, MERS and 2019-nCoV.

The acylated spiramycin compounds or the compositions thereof according to the present disclosure bind to a main protease Mpro inhibition site of coronavirus.

The acylated spiramycin compounds or the compositions thereof according to the present disclosure bind to coronavirus S protein and its host cell receptor ACE2 protein.

In particular, isovalerylspiramycin I, isovalerylspiramycin II, isovalerylspiramycin III or carrimycin according to the present disclosure binds to coronavirus S protein and its host cell receptor ACE2 protein.

In particular, isovalerylspiramycin I, isovalerylspiramycin II, isovaleryl spiramycin III or carrimycin according to the present disclosure binds to a coronavirus main protease Mpro inhibition site.

The medicine in the present disclosure comprises one of acylated spiramycin compounds, or a combination of at least two of acylated spiramycin compounds, or carrimycin as a first active pharmaceutical ingredient, with a second active pharmaceutical ingredient for supplementation; the second active pharmaceutical ingredient can be selected from antiviral medicine and/or anti-AIDS medicine. The first active pharmaceutical ingredient and the second active pharmaceutical ingredient can be prepared separately or compounded into one preparation.

Preferably, one of isovalerylspiramycin I, isovalerylspiramycin II, isovalerylspiramycin III or a combination of at least two of them or carrimycin according to the present disclosure is used as the first active pharmaceutical ingredient to be combined with the second active pharmaceutical ingredient.

The second active pharmaceutical ingredient can be a protease inhibitor, a fusion protein inhibitor, a nucleoside reverse transcriptase inhibitor, an immunosuppressant, etc., and is selected from at least one of the following active pharmaceutical ingredients: Indinavir, Saquinavir, Lopinavir, Carfilzomib, ritonavir, Remdesivir or Remdesivir (RDV, GS-5734), Atazanavir, Darunavir, Tipranavir, Fosamprenavir, Enzaplatovir, Presatovir, Ribavirin, Abacavir, Bortezomib, Elvitegravir, Montelukast, Deoxyrhapontin, Polygonin, Sophoradin, Disulfiram, Carmofur, Shikonin, Ebselen, Tideglusib, PX-12, TDZD8, Cinanserin, Cyclosporin A, Albuvirtide, Ambroxol, Interferon, Abidor, Oseltamivir and other medicine or active ingredients in medicine.

The specific embodiments of the present disclosure will be described in further detail with reference to the accompanying drawings.

## BRIEF DESCRIPTION OF DRAWINGS

The attached drawings, as a part of the present disclosure, are used to provide a further understanding for the present disclosure. The illustrative embodiments of the present disclosure and their descriptions are used to explain the present disclosure, but do not constitute an improper limitation of the present disclosure.

Fig. 1 is one of the schematic diagrams of the combination of isovalerylspiramycin I and angiotensin converting enzyme ACE2 in the present disclosure;

Fig. 2 is the second schematic diagram of the combination of isovalerylspiramycin I and angiotensin converting enzyme ACE2 in the present disclosure;

Fig. 3 is a schematic diagram of the combination of isovalerylspiramycin I and spike protein in the present disclosure;

Fig. 4 is a schematic diagram of the 3D structure of SARS-CoV main protease (Mpro, also known as 3CL protease) and its inhibitor binding site, that is, its function in mediating virus replication and transcription;

Fig. 5 is a schematic diagram of the combination of isovalerylspiramycin I and 3CL in the present disclosure;

Fig. 6 shows the docking of several different medicines with SARS-CoV Mpro;

Fig. 7 is a schematic diagram of inhibition of CCR5 expression by carrimycin;
Fig. 8 is a schematic diagram of a systematic flexibility analysis of isovalerylspiramycin I (CM926) and SARS-S;
Fig. 9 is a schematic diagram showing the combination of isovalerylspiramycin I (CM926) and SARS-S; (A) 2D binding mode of SARS-S and CM926; (B) a binding model of CM926 on the SARS-S molecular surface; (C) a 3D binding mode of SARS-S and CM926;
Fig. 10 shows a systematic flexibility analysis of isovalerylspiramycin I (CM926) and SARS-M;
Fig. 11 is a schematic diagram showing the combination of isovalerylspiramycin I (CM926) and SARS-M; (A) a 2D binding mode of SARS-M and CM926; (B) a binding model of CM926 on the SARS-M molecular surface; (C) a 3D binding mode of SARS-M and CM926;
Fig. 12 shows systematic flexibility analysis of isovalerylspiramycin I (CM926) and ACE2;
Fig. 13 shows the combination of isovalerylspiramycin I(CM926) and ACE2; (A) a 2D binding mode of ACE 2 and CM926; (B) a binding model of CM926 on ACE2 molecular surface; (C) a 3D binding mode of ACE2 and CM926;
Fig. 14 shows systematic flexibility analysis of isovalerylspiramycin I (CM926) and ACE2-SARS;
Fig. 15 is a schematic diagram showing the combination of isovalerylspiramycin I (CM926) and ACE2-SARS, (A) a 2D binding mode of ACE2-SARS and CM926; (B) a binding model of CM926 on ACE2-SARS molecular surface; (C) a 3D binding mode of ACE2-SARS and CM926;
Fig. 16 shows the inhibitory effect of carrimycin on the CPE induced by an HCoV-229E strain in Huh 7.5 cells;
Fig. 17 shows the anti-inflammatory and immunomodulation-inhibition of PI3K/Akt/mTOR pathway by carrimycin;
Fig. 18 shows that carrimycin can significantly reduce lung injury;
Fig. 19 shows one of the improvements of lung inflammation before and after treatment with carrimycin;
Fig. 20 shows another improvement of lung inflammation before and after treatment with carrimycin;
Fig. 21 shows the improvement of lung inflammation of a patient in case 1 before and after treatment with carrimycin in specific cases.

## SPECIFIC EMBODIMENTS

In order to make the objectives, technical solutions and advantages of the embodiments of the present disclosure clearer, the technical solutions in the embodiments will be described clearly and completely in conjunction with the drawings in the embodiments of the present disclosure. The following embodiments are used to illustrate the present disclosure, but are not intended to limit the scope of the present disclosure.

Computer simulation experiment:

Referring to Fig. 2, isovalerylspiramycin I is one of the main active substances and one of the characteristic ingredients of carrimycin. The molecular docking showed that isovalerylspiramycin I could bind with a spike-ACE2 protein complex at multiple sites. Isovalerylspiramycin I has strong binding with ACE2, and the binding position is located in the pocket of ACE2 active site (Fig. 1). This structure is related to the catalytic function of ACE2. When ACE2 plays a catalytic role, the three-dimensional structure of this area (ACE2) needs to be changed. The binding of isovalerylspiramycin I with ACE2 will limit the three-dimensional structure change of ACE2, so isovalerylspiramycin I has an inhibitory effect on ACE2. Isovalerylspiramycin compounds II and III also bind to spike protein, and the binding position are located in the pocket of ACE2 active site (omitted in the figures).

The S proteins (spike) of coronaviruses are combined into a trimer, which contains about 1300 amino acids and belongs to Class I viral fusion proteins. Similar virus membrane fusion proteins include Env proteins of HIV, HA proteins of influenza, Gp proteins of Ebola virus and so on. The S protein determines the host range and specificity of the virus, and is also an important action site for neutralizing antibodies in a host. Figs. 3a and 3b show that isoamylspiramycin I also binds to the site near an RBD domain of the spike protein, so isoamylspiramycin I is likely to inhibit the binding of SARS viruses to the host receptor. It indicates that valerylspiramycin I according to the present disclosure can inhibit SARS viruses or influenza viruses. Isovalerylspiramycin compounds II and III also bind to spike proteins, so isovalerylspiramycin compounds II and III or carrimycin can inhibit SARS viruses or influenza viruses.

On January 21, 2020, Chinese scientists (researcher Hao Pei of Institut Pasteur of Shanghai, Chinese Academy of Sciences, researcher Zhong Wu of National Center for Emergency Prevention and Control Medicine Engineering Technology, Academy of Military Medical Sciences, and researcher Li Xuan of Key Laboratory of Synthetic Biology, Center of Excellence in Molecular Plants, Chinese Academy of Sciences) published online a paper entitled "Evolution of the novel coronavirus from the going Wuhan outbreak and modeling of its spike protein for risk of human transmission" in SCIENCE CHINA Life Sciences (English version). By comparing the genome of the novel coronavirus SARS-CoV-2 with that of SARS coronavirus in 2002 and MERS coronavirus, it was found that there were ~70% and ~40% sequence similarities on average, among which the key spike gene (coded S-protein) between different coronaviruses and host cells had greater differences. The novel coronavirus SARS-CoV-2 was found to belong to Betacoronavirus. Betacoronavirus is a protein-coated single-stranded positive-stranded RNA virus, which parasitizes and infects higher animals (including humans). In the position of evolutionary tree, SARS-CoV-2 is adjacent to SARS (causing SARS in 2002) virus and SARS-like virus, but it does not belong to SARS and SARS-like virus group; evolutionary common outgroup is an HKU9-1 coronavirus parasitic on fruit bats. In this paper, the structural docking between the S-protein of the novel coronavirus SARS-CoV-2 and human ACE2 protein was studied by using the calculation method of molecular structure simulation, and surprising results were obtained. Although four of the five key amino acids in the S-protein of the novel coronavirus SARS-CoV-2 binding to ACE2 protein have changed, the changed amino acids perfectly maintain the original structural conformation of the interaction between the S-protein of SARS virus and ACE2 protein as a whole. Although the interaction between the new structure of the novel coronavirus SARS-CoV-2 and ACE2 protein has decreased due to a few missing hydrogen bonds (compared with the interaction between S-protein of SARS virus and ACE2), it still has a strong binding free energy (-50.6 kcal/mol). The results indicate that the novel coronavirus SARS-CoV-2 infects human respiratory epithelial cells through the molecular mechanism of interaction between S-protein and human ACE2. The S-protein of SARS and the S-protein of 2019-nCoV have almost the same 3D structure in the RBD domain, which directly interacts with the host cell receptor ACE2. Based on the S-protein structure of SARS, the structure of 2019-nCoV was simulated, and then the ability of interaction between S-protein of 2019-nCoV and human ACE2 molecule was evaluated based on the structure of a composite crystal composed of SARS and ACE2. The free energy of binding between S-protein of 2019-nCoV and human ACE2 is -50.6 kcal/mol, which is 28 kcal/mol higher than -78.6 kcal/mol of SARS. In comparison, the binding of S- protein of 2019-nCoV and human ACE2 is relatively weak, but this affinity is still considered to be very strong.

The results of molecular simulation showed that isovalerylspiramycin I, one of the main active ingredients of carrimycin, binds to the SARS-CoV S protein and its host cell receptor ACE2 protein, and had a strong binding effect on the latter.

The binding of isovalerylspiramycin I and ACE2 protein will limit the three-dimensional structure changes of ACE2, indicating that isovalerylspiramycin I has potential ACE2 inhibitory activity. SARS infection will lead to a decrease in ACE2 expression, which in turn leads to increased lung damage. Based on the fact that the binding of small molecules and proteins can enhance protein stability, the binding of isovalerylspiramycin I and ACE2 can inhibit the function of ACE2 on the one hand and increase the stability of ACE2 and on the other hand, and it is possible to inhibit the decrease of ACE2 expression after SARS infection. ACE2 is expressed in human cornea and conjunctiva. There have been reports of infection of 2019-nCoV through cornea and conjunctiva, which further proves that 2019-nCoV infects human respiratory epithelial cells through the interaction of S-protein and human ACE2. Isovalerylspiramycin I can have an inhibitory effect on both the coronavirus S protein and its host cell receptor ACE2, so it can have an inhibitory effect on coronavirus infection. Isovalerylspiramycin III and isovaleryl Spiramycin II having a similar structure with isovalerylspiramycin I, butyrylspiramycin III, propionylspiramycin III, acetylspiramycin III, butyrylspiramycin II, propionylspiramycin II, acetylspiramycin II and other active ingredients of carrimycin as well as carrimycin have potential inhibitory effect on coronavirus infection, especially for 2019-nCoV infection.

Fig. 4 shows that SARS-CoV main protease (Mpro), also known as 3CL protease, plays a key role in mediating virus replication and transcription. The binding site of the inhibitors is between domain I and domain II.

Fig. 5 shows that the binding site of isovalerylspiramycin I and 3CL is consistent with that of the 3CL inhibitors. Mpro (3CL) has been used in the screening model of coronavirus medicines by molecular simulation technology in Shanghai Institute of Meteria Medica, Chinese Academy of Sciences, and the relevant screening results were released on January 25, 2020. Therefore, isovalerylspiramycin I has a great potential value in anti-viral infection. Isovalerylspiramycin III, isovalerylspiramycin II having a similar structure with isovalerylspiramycin I, butyrylspiramycin III, propionylspiramycin III, acetylspiramycin III, butyrylspiramycin II, propionylspiramycin II, acetylspiramycin II and other active ingredients of carrimycin, as well as carrimycin have a great potential value in anti-viral infection, especially in 2019-nCoV infection.

The present disclosure also compares the docking of several different medicines with SARS-CoV Mpro, as shown in Fig. 6 and Table 1. It can be seen that, among these medicines, the docking of isovalerylspiramycin I and SARS-CoV Mpro is obviously stronger than other medicines, not only superior to similar medicines, but also superior to lopinavir and ritonavir with antiviral effects. However, other antibiotics commonly used in clinic not only have weaker binding force than isovalerylspiramycin I, but also cannot completely bind to the inhibition site, which makes it difficult to effectively inhibit Mpro. Isovalerylspiramycin I can perfectly completely bind to the Mpro inhibition site because of its isovaleryl group, which indicates that isovalerylspiramycin I, isovalerylspiramycin III, isovalerylspiramycin II, butyrylspiramycin III, propionylspiramycin III, acetylspiramycin III, butyrylspiramycin II, propionylspiramycin II, acetylspiramycin II and other active ingredients of carrimycin, as well as carrimycin have a good clinical value in coronavirus infection, inflammatory reaction and secondary infection.

**Table 1 Binding energy of several different medicines to SARS-CoV $M^{pro}$**

| | FullFitness ( kcal/mol ) | deltaG ( kcal/mol ) |
|---|---|---|
| Isovalerylspiramycin I | -1137.97 | -12.871312 |
| Spiramycin I | -1144.6531 | -9.870569 |
| Azithromycin | -1113.8289 | -9.9098425 |
| Erythromycin | -1117.0308 | -9.550697 |
| Moxifloxacin | -999.2253 | -8.187792 |
| Acyclovir | -1398.953 | -7.08006 |
| Ritonavir | -1302.054 | -9.768603 |
| Lopinavir | -1235.8148 | -9.789774 |
| CMK inhibitor | -1209.9166 | -10.398779 |
| Clarithromycin | -1140.7974 | -10.516921 |
| Cinanserin | -1260.691 | -8.871793 |
| Carbapenem | -1154.617 | -6.569598 |

The above results indicate that isovalerylspiramycin I, spiramycin I, azithromycin, erythromycin, moxifloxacin, clarithromycin and other antibiotics, as well as macrolide antibiotics have certain effects, and can become potential medicines for treating novel coronavirus infection.

At present, the main reported symptoms of novel virus infection are interstitial pneumonia caused by immune injury reaction, multiple organ dysfunction, lung injury, etc. Although the mechanism of the curative effect of carrimycin observed from previous experiments remains to be further clarified, in view of the fact that the novel pneumonia itself is complicated with bacterial infection and the safety of carrimycin is high, carrimycin can be used to treat novel virus infection.

Furthermore, isovalerylspiramycin I and proteins such as SARS-Spike, SARS-Mpro, ACE2, ACE2-SARS- Spike are used for molecular docking respectively, and then molecular dynamics simulation is carried out.

Molecular docking method:

MOE software is used for molecular docking. Energy optimization is carried out on isovalerylspiramycin I (small-molecule CM926) in MOE to obtain low-energy three-dimensional conformations as ligands. The definition of binding sites is as follows: SARS-S (Cys323, Ser358), SARS-M (Glu166, Phe140, Cys145), ACE2 (Lys74, Ser106, Gln102, Asp350), ACE2-SARS (Asp38, Gln42, Gln325, Glu329, Arg426, Tyr436). An AMBER10:EHT force field and an R-field implicit solvent model are selected before formal docking. The docking process adopts a flexible induced fit mode, and the side chain binding to pocket amino acids can be optimized and adjusted according to the ligand conformation, and the weight of restricting the rotation of the side chain is set to 10. The binding modes of ligands are sorted by London dG scoring function, and the binding free energy of the first 30 conformations is evaluated again by further optimization and GBVI/WSA dG method. The binding model with the best score (lowest binding free energy) is selected for molecular dynamics simulation.

Molecular dynamics simulation

An MMFF94x force field is selected to generate the parameters of small-molecule isovalerylspiramycin I. Hydrogen atoms of isovalerylspiramycin I are optimized by Gaussian09 software package under HF/6-31g*. The charge is calculated by HF/6-31g*, and the electrostatic potential is fitted by an RESP4 fitting method. Sodium/chlorine counterions are added into the complex to neutralize the system, and then solvated in the rectangular water box of TIP3P, so that a solvent layer of 10 Å is formed between the edge of the water box and the solute surface.

All molecular dynamics simulations are carried out in AMBER16 software. Sodium/chlorine counterions are added into the complex to keep the system electrically neutral. AMBER GAFF and FF14SB force fields are applied, and the SHAKE algorithm is used to limit all hydrogen atoms involved in covalent bonds. The time step is 2 fs. A particle mesh Ewald (PME) method is used to deal with long-range electrostatic interaction. Two energy optimization steps are performed for each solvation system before the heating step. The first 4000 steps of energy optimization are carried out under the condition that all heavy atoms are limited by 50 kcal/( mol·Å2), while solvent molecules and hydrogen atoms could move freely. Then unconstrained energy optimization is carried out, including 2000 steps of steepest descent optimization and 2000 steps of conjugate gradient optimization. Then, under the condition of constant volume, the whole system is heated from 0 K to 300 K in 50 ps, and then simulation is carried out for 400 ps under the constant pressure

of 1 atm by using Langevin dynamics. In the heating step, a weak energy constraint of 10 kcal/ (mol·Å2) is used to limit all heavy atoms. In the equilibrium stage of dynamic simulation, the periodic boundary is adopted and the simulation is carried out for 20 ns under NPT (constant composition, pressure and temperature) conditions (constant pressure of 1 atm and 300 K). Finally, the binding free energy of the composite is calculated by an MM-PBSA method.

As shown in Fig. 8, the RMSD value of the skeleton atom of the SARS-S protein is less than 2.5 angstrom, and the RMSD value of isovalerylspiramycin I (small molecule CM926) is less than 5.0 angstrom, so the whole system reaches equilibrium within the simulation time, indicating that the force field used in system simulation is appropriate. Cluster analysis is carried out on the trajectory obtained by dynamic simulation, and the cluster center of the trajectory after system stabilization is taken as the final binding mode conformation of isovalerylspiramycin I and the protein SARS-S, as shown in Fig. 9.

Isovalerylspiramycin I is located around the binding site of SARS-S. However, there is no obvious hydrogen bond or Pi-Pi stacking interaction between isovalerylspiramycin I and SARS-S. Most of isovalerylspiramycin I is exposed to solvent. The Van der Waals interaction is formed between CM926 and the residues around the binding site.

As shown in Fig. 10, the RMSD value of protein SARS-M skeleton atom is less than 2.5 angstrom, and the RMSD value of small-molecule CM926 is less than 3.0 angstrom, so the whole system reaches equilibrium within the simulation time, indicating that the force field used in system simulation is appropriate. Cluster analysis is carried out on the trajectory obtained by dynamic simulation, and the cluster center of the trajectory after the system stabilization is taken as the final binding mode conformation of small molecule CM926 and protein SARS-M, as shown in Fig. 11.

The binding sites of CM926 and SARS-M form proper spatial complementarity. There are hydrogen bonds and electrostatic interactions between CM926 and SARS-M. The oxygen atom in the hydroxyl group of CM926, which is regarded as a hydrogen bond donor, forms a hydrogen bond with the oxygen atom in the side chain of Glu166. The nitrogen atom in the amino group of CM926 is regarded as a hydrogen bond donor, and forms hydrogen bonds with the oxygen atoms in the side chains of Glu166 and Gln189, respectively. The positively charged nitrogen atom in the amino group forms electrostatic interaction with the negatively charged oxygen atom in the side chain of Glu166. A VDW interaction is formed between CM926 and the surrounding residues. These interactions promote the binding between CM926 and SARS-M protein.

As shown in Fig. 12, the RMSD value of the skeleton atom of protein ACE2 is less than 3.0 angstrom, and the RMSD value of the small-molecule CM926 is less than 3.0 angstrom, and the whole system reaches equilibrium within the simulation time, which indicates that the force field used in the system simulation is appropriate. Cluster analysis is carried out on the trajectory obtained by dynamic simulation, and the cluster center of the trajectory after system stabilization is taken as the final binding mode conformation of small-molecule CM926 and protein ACE2, as shown in Fig. 13.

The binding sites of CM926 and ACE2 form proper spatial complementarity. There are hydrogen bonds and electrostatic interactions between CM926 and ACE2. The oxygen atom in the hydroxyl group of CM926 is regarded as a hydrogen bond donor and forms a hydrogen bond with the oxygen atom in the side chain of Asp206. The nitrogen atom in the amino group of CM92 is regarded as a hydrogen bond donor, and forms hydrogen bonds with the oxygen atoms in the side chains of Asp206 and Asp350, respectively. The oxygen atom in the carbonyl group of CM926 is regarded as a hydrogen bond acceptor and forms a hydrogen bond with the nitrogen atom in side chain of Lys562. The positively charged nitrogen atoms in amino groups form electrostatic interactions with the negatively charged oxygen atoms in the side chains of Asp206 and Asp350, respectively. A VDW interaction is formed between CM926 and the surrounding residues. The main interactions promote the binding between CM926 and protein ACE2.

As shown in Fig. 14, the RMSD value of the skeleton atom of the protein ACE2-SARS is less than 5.0 angstrom, and the RMSD value of small-molecule CM926 is less than 3.0 angstrom, so the whole system reaches equilibrium within the simulation time, which indicates that the force field used in system simulation is appropriate. Cluster analysis is carried out on the trajectory obtained by dynamic simulation, and the cluster center of the trajectory after system stabilization is taken as the final binding mode conformation of small-molecule CM926 and protein ACE2-SARS, as shown in Fig. 15.

The binding sites of CM926 and ACE2-SARS form proper spatial complementarity. There are hydrogen bonds and electrostatic interactions between CM926 and ACE2-SARS. The nitrogen atom in the amino group of CM926A is regarded as a hydrogen bond donor, and forms a hydrogen bond with the oxygen atom in the side chain of Glu35 of ACE2 subunit in a complex protein. The positively charged nitrogen atom in the amino group forms electrostatic interaction with the negatively charged oxygen atom in the side chain of Glu35. A VDW interaction is formed between CM926 and the surrounding residues. These interactions promote the binding between CM926 and ACE2-SARS.

The free energy ΔGtotal of binding between CM926 and protein is shown in Table 1. The free energy of binding consists of four parts, in which Van der Waals interaction energy term is expressed by ΔEvdw, electrostatic energy term is expressed by ΔEele, the polar interaction energy term of the solvent is expressed by ΔGpolar, and nonpolar interaction energy term of the solvent is expressed by ΔGnonpolar. The free energy of binding between small-molecule CM926 and proteins SARS-S, SARS-M, ACE2 and ACE2-SARS are -3.26, -6.20, -9.16 and -6.76 kcal/mol, respectively. The predicted approximate IC50 value is calculated by the following formula:

$$\Delta Gtotal \approx RTlnIC50$$

where, R is 8.314 and T is 300K.

The final predicted approximate IC50 values are 1817.33μM, 30.27μM, 0.21μM and 11.82μM, respectively.

Table 2 Average Binding Energy and its Components Obtained from the MM-PB SA Calculation for the complexes

| Contribution | Energy (kcal/mol) | | | |
|---|---|---|---|---|
| | SARS-S | SARS-M | ACE2 | ACE2-SARS |
| $\Delta E_{vdw}$ | -3.26 | -4.18 | -8.49 | -5.74 |
| $\Delta E_{ele}$ | 14.83 | -27.99 | -81.63 | -37.31 |
| $\Delta G_{polar}$ | -12.79 | 29.03 | 86.87 | 40.02 |
| $\Delta G_{nonpolar}$ | -2.54 | -3.06 | -5.91 | -3.73 |
| $\Delta G_{total}$ | -3.76 | -6.20 | -9.16 | -6.76 |

By a molecular dynamics simulation experiment, the inhibitory effects of isovalerylspiramycin I on the RBDs of the SARS Mpro protein, the ACE2 protein and the SARS Spike protein are predicted, and their IC50 values are about 30.27μM, 0.21μM and 11.82μM, respectively. Therefore, it is speculated that carrimycin has great application value in inhibiting novel coronavirus (2019-nCoV) infection and virus replication.

In vitro experiments

According to the disclosure, in vitro experiments are carried out on carrimycin, isovalerylspiramycin I, isovalerylspiramycin II and isovaleryl spiramycin III, respectively, to verify their effects on the coronavirus. And a cytopathic effect (CPE) experiment is adopted to measure the half-maximal inhibitory concentration ($IC_{50}$) and SI on coronaviruses (HCoV-229E and HCoV-OC43) in Huh7.5 cells, HCT-8 cells and MRC-5 cells.

Materials

Test product: carrimycin, provided by Shenyang Tonglian Group Co., Ltd.. 20mg/ml of mother liquor was prepared from DMSO and stored in a refrigerator at 4DEG C. During the experiment, the culture solution was diluted to an appropriate concentration, and stored in a refrigerator at 4DEG C.

Positive control medicine: Ribavirin Injection (RBV) was purchased from Tianjin KingYork Group Hubei Tianyao Pharmaceutical Co., Ltd., with the batch number of 31712252 and the specification of 100mg/ml. It was diluted to the required concentration and stored in a refrigerator at 4DEG C.

Azithromycin: commercially available

Cells

The subcultured human colon cancer cell HCT-8, human liver cancer cell Huh7.5 and human diploid cell MRC-5 were all subcultured in our laboratory (Institute of Livelihood, Chinese Academy of Sciences) and cultured in a DMEM or 1640 medium containing 10% inactivated fetal bovine serum and 1% double antibodies (penicillin and streptomycin) at 37DEG C in a 5% $CO_2$ incubator. Subculture is carried out once every 2-3 days.

HCoV-229E was subcultured in Huh7.5 cells and stored in a refrigerator at -80DEG C. HCoV-OC43 was subcultured in HCT-8 cells and stored in a refrigerator at -80DEG C.

The DMEM liquid medium, 1640 liquid medium, fetal bovine serum, the penicillin-streptomycin solution, PBS (PH=7.4) and 0.25% Trypsin-EDTA were all purchased from Invitrogen Company.

Experimental supplies and instruments: a cell culture bottle, a 96-well culture plate and a pipette were products of Corning Incorporated of the United States; a carbon dioxide incubator (Model 3111) was a product of Thermo Corporation of the United States; a biosafety cabinet was a product of American NUAIRE Company; an inverted microscope was a product of Olympus Corporation; a vacuum pump was a product of INTEGRA Biosciences Corporation; a 12-channel pipette and a single-channel pipette are Eppendorf products.

Experimental method: taking HCT-8 cells as an example, 3 ml of 0.25% Trypsin-EDTA was added into a culture bottle full of HCT-8 cells and digested at 37DEG C for 1-2 minutes, the digestive juice was discarded, a culture solution

was added, blown, and subcultured with 1:4 once every 2-3 days. 200,000 cells per milliliter were prepared during inoculation onto a plate, and were inoculated in a 96-well cell culture plate with 0.1ml for per well, and culture was carried out at 37DEG C in a 5% $CO_2$ incubator overnight, and experiments were carried out after the cells grew into a monolayer.

The activity determination of anti-HCoV-229E (by the CPE method): the experiment was carried out in subcultured Huh7.5 cells. Huh7.5 cells were inoculated in a 96-well plate with $1\times10^4$ cells per well. After overnight culture, 100μl of HCoV-229E virus solution was infected with Huh7.5 cells in the 96-well plate, and the drug to be determined was diluted with a maintenance solution, and was determined by adopting two administration schemes including administration at the same time of infection and administration 2 hours after infection, respectively. The drug to be determined was diluted three times into 8 doses of samples, and each dose was provided with 2 parallel wells. When the lesion of a virus control group reached 4+, the results were observed, and a Reed-Muench method was used to calculate the half-maximal inhibitory concentration (the formula is as follows) and the selection index (SI= IC50/ TC50) of the drug to the virus.

$$IC_{50} = \mathrm{AntiLog}\left(A+\frac{B-50}{50-C}\times D\right)$$

where: A= a drug concentration with a cumulative inhibition rate less than 50%, B=an inhibition rate with the cumulative inhibition rate greater than 50%, C= an inhibition rate with the cumulative inhibition rate less than 50%, D = log dilution multiple

Experimental results

Inhibitory effect of the drug on HCoV-229E in Huh7.5 cells

As shown in Table 3, administration at the same time of infection: the IC50 of 0h administration of carrimycin on an HCoV-229E strain was 3.14±0.80 μg/ml, and the selection index SI was 13.0; the IC50 of 0h administration of RBV on the HCoV-229E was 7.06±0.91 μg/ml, and the selective index SI was greater than 14.2; the IC50 of 0h administration of azithromycin on the HCoV-229E was 16.78±3.48 μg/ml, and the selective index SI was 5.0, as determined by the CPE method.

Administration 2 hours after infection: the IC50 of 2h administration of carrimycin on the HCoV-229E strain was 2.36±0.30 μg/ml, and the selection index SI was 15.1; the IC50 of 2h administration of RBV on the HCoV-229E was 6.24±2.07 μg/ml, and the selective index SI was greater than 16.0; the IC50 of 2h administration of azithromycin on HCoV-229E was 9.55±6.75 μg/ml, and the selective index SI was 6.7, as determined by the CPE method.

Table 3: Inhibitory effects ($IC_{50}$) of medicine on HCoV-229E in Huh7.5 cells (by the CPE method)

| | carrimycin (μg/ml) | | azithromycin (μg/ml) | | RBV (μg/ml) | |
|---|---|---|---|---|---|---|
| | 0h | 2h | 0h | 2h | 0h | 2h |
| First batch | 2.57 | 2.57 | 14.32 | 14.32 | 6.42 | 7.70 |
| Second batch | 3.70 | 2.14 | 19.24 | 4.77 | 7.70 | 4.77 |
| Mean value | 3.14±0.80 | 2.36±0.3 0 | 16.78±3.4 8 | 9.55±6.7 5 | 7.06±0.91 | 6.24±2.0 7 |
| SI | 13.0 | 15.1 | 5.0 | 6.7 | >14.2 | >16.0 |

Curative effects of drugs against an HCoV-OC43 strain

As shown in Table 4: in HCT-8 cells, the IC50 of 0h administration of carrimycin on an HCoV-OC43 strain was 25.87 μg/ml, and the selection index SI was 2.2; the IC50 of 0h administration of RBV on the HCoV-OC43 was 11.11 μg/ml, and the selective index SI was greater than 9.0; 0h administration of azithromycin had no inhibitory effect on the HCoV-OC43, as determined by the CPE method.

As shown in Table 5: in MRC-5 cells, the IC50 of 0h administration of carrimycin on the HCoV-OC43 strain was 5.93 μg/ml, and the selection index SI was 3.2; the IC50 of 0h administration of RBV on the HCoV-OC43 was 63.75 μg/ml, and the selective index SI was greater than 1.6; 0h administration of azithromycin had no inhibitory effect on the HCoV-OC43, as determined by the CPE method.

Table 4: Inhibitory effects ($IC_{50}$) of medicines on HCoV-OC43 in HCT-8 cells (by the CPE method)

| Compound | $TC_{50}$ (μg/ml) | $IC_{50}$ (μg/ml) | SI |
|---|---|---|---|
| azithromycin | >100 | >100 | - |
| carrimycin | 57.74 | 25.87 | 2.2 |
| RBV | >100 | 11.1 | >9.0 |

Table 5: Inhibitory effects ($IC_{50}$) of medicines on HCoV-OC43 in MRC-8 cells (by the CPE method)

| Compound | $TC_{50}$ (μg/ml) | $IC_{50}$ (μg/ml) | SI |
|---|---|---|---|
| azithromycin | 48.07 | >11.1 | - |
| carrimycin | 19.25 | 5.93 | 3.2 |
| RBV | >100 | 63.75 | >1.6 |

Fig. 16 shows the inhibitory effect of carrimycin on the CPE induced by the HCoV-229E strain in Huh 7.5 cells

Summary:

Under the experimental conditions: in Huh7.5 cells, the IC50 of 0h administration of carrimycin on the HCoV-229E strain was 3.14±0.80 μg/ml, and the selection index SI was 13.0; the IC50 of 0h administration of RBV on the HCoV-229E was 7.06±0.91 μg/ml, and the selective index SI was greater than 14.2; the IC50 of Oh administration of azithromycin on HCoV-229E was 16.78±3.48 μg/ml, and the selective index SI was 5.0, as determined by the CPE method. The IC50 of 2h administration of carrimycin on the HCoV-229E strain was 2.36±0.30 μg/ml, and the selective index SI was 15.1; the IC50 of 2h administration of RBV on the HCoV-229E was 6.24±2.07 μg/ml, and the selective index SI was greater than 16.0; the IC50 of 2h administration of azithromycin on the HCoV-229E was 9.55±6.75 μg/ml, and the selective index SI was 6.7, as determined by the CPE method.

In HCT-8 cells, the IC50 of 0h of administration of carrimycin on the HCoV-OC43 strain was 25.87 μg/ml, and the selection index SI was 2.2; the IC50 of 0h of administration of RBV on the HCoV-OC43 was 11.11 μg/ml, and the selective index SI was greater than 9.0; 0h of administration of azithromycin had no inhibitory effect on the HCoV-OC43, as determined by the CPE method.

In MRC-5 cells, the IC50 of 0h administration of carrimycin on the HCoV-OC43 strain was 5.93 μg/ml, and the selection index SI was 3.2; the IC50 of 0h administration of RBV on the HCoV-OC43 was 63.75 μg/ml, and the selective index SI was greater than 1.6; 0h of administration of azithromycin had no inhibitory effect on HCoV-OC43, as determined by the CPE method.

The following conclusions can be drawn from the above experiments:
under the experimental conditions, carrimycin had inhibitory effect on both the HCoV-OC43 strain and the HCoV-229E strain; RBV had inhibitory effect on both the HCoV-OC43 strain and the HCoV-229E strain; azithromycin had inhibitory effect on the HCoV-229E strain, but no inhibitory effect on the HCoV-OC43 strain. Under the experimental conditions, carrimycin has definite in-vitro antiviral activity against HCoV-229E and HCoV-OC43, and is superior to azithromycin. The antiviral activity of RBV against HCoV-229E and HCoV-OC43 is equivalent to the results in literatures and before this experiment, which indicates that the experimental system is established.

The above results indicated that the curative effect of carrimycin against the two viruses was stronger than that of ribavirin, a control drug, and azithromycin, a macrolide antibiotic, which indicated that carrimycin had good anti-coronavirus activity and had a good application prospect.

The present disclosure adopts the above method to detect isovalerylspiramycin I, isovalerylspiramycin II and isovalerylspiramycin III, wherein the results showed that the inhibition activity of isovalerylspiramycin I is higher than the inhibition activity of carrimycin, and the inhibition activities of isovalerylspiramycin II and isovalerylspiramycin III are close to the inhibition activities of carrimycin.

In addition, the present disclosure also adopts the above-mentioned method to carry out the in-vitro experiments to prove that carrimycin, isovalerylspiramycin I, isovalerylspiramycin II and isovalerylspiramycin III inhibit the Middle East Respiratory Syndrome Coronavirus (MERS-CoV), the severe acute respiratory syndrome coronavirus SARS-CoV, the human coronavirus HKU1 and the human coronavirus NL63(HCoV-NL63), and the results showed that the inhibition activities of the four medicines are all higher than the inhibition activity of ribavirin or azithromycin.

Particularly, in vitro experiments on the anti-2019-nCoV activities of carrimycin, isovalerylspiramycin I, isovalerylspiramycin II and isovalerylspiramycin III are carried out as follows:

Tested medicines: carrimycin, isovalerylspiramycin I, isovalerylspiramycin II and isovalerylspiramycin III.
Cells: VeroE6 cells, which were preserved by Pathogen Center, Institute of Medical Experimental Animals, Chinese Academy of Medical Sciences.
Viruses: 2109-nCoV, with a titer of 105TCID50/ml, stored at -80DEG C by the Pathogen Center, Institute of Medical Experimental Animals, Chinese Academy of Medical Sciences. The titer of the viruses used was 100TCID50.

Experimental method:

Tested medicines: two concentrations of carrimycin: 10 μg/ml and 2 μg/ml were used in the experiment.

Table 6 Drug Name and Concentration

| Name of tested drug | Concentration (two concentrations were set for each drug) |
|---|---|
| carrimycin | 10 μg/ml, 2 μg/ml |

Antiviral experiment of tested medicines

(1) a sterile 96-well culture plate was used, 200μl Vero E6 cells with a concentration of $5\times10^4$cell/ml was added to each well, and cultured at 37DEG C in a 5% $CO_2$ incubator for 24 hours;
(2) the tested drug was diluted into two concentrations, each concentration for 5 multiple wells, each well was filled with 100μl, and then 100 TCID50 virus of equal volume was added to each well for reaction for 1 hour;
(4) after 1 hour, the cell culture solution in the 96-well culture plate was discarded and the above mixed solution was added;
(5) a cell control, a blank control (a solvent control) and a virus control (negative control) are established at the same time;
(6) the cells were incubated in a 5% $CO_2$ incubator at 37DEG C for 4-5 days;
(7) cytopathic effect (CPE) was observed under an optical microscope, and complete lesion was recorded as "+++", 75% lesions as "++", 50% lesions as "++", 25% lesions as "+", and no lesions as "-".

Experimental conditions: all the above experimental operations were completed in a BSL-3 laboratory.

Result judgment:

The absence of CPE in cells indicated an effective concentration to inhibit viruses, while the presence of CPE indicated an ineffective concentration.

Experimental results:

The replication of 2019-nCoV can be effectively inhibited by setting two concentrations for the drug, and the results are shown in Table 7.

Table 7 Effect of carrimycin against 2019-nCoV

| Name of tested drug | Concentration | Result |
|---|---|---|
| carrimycin | 10 μg/ml | - |
| | 2 μg/ml | - |
| **Negative control** | | ++++ |

Conclusion: according to the screening results at the cell level, carrimycin can inhibit the replication of 2109-nCoV in cells at the concentration of 2 μg/ml, suggesting that carrimycin has an anti-2109-nCoV activity in vitro.
Isovalerylspiramycin I, isovalerylspiramycin II and isovalerylspiramycin III also have anti-2109-nCoV activities in vitro.

$IC_{50}$ calculation of carrimycin

Table 8

| Drug name | Carrimycin | Dilution multiple | 4 | Virus name: | 2019-nCoV | | | | Use | Treatment | IC50= | 0.99 |
|---|---|---|---|---|---|---|---|---|---|---|---|---|
| Concentration (ug/ml) | First row | Second row | Third row | Normal control | Virus control | | | | Average | Accumulative | 1-accumulative | Cumulative inhibition rate% |
| 10.00 | 4 | 4 | 4 | 4 | 0 | 1.000 | 1.000 | 1.000 | 1.00 | 2.25 | 0.00 | 1.0000 |
| 2.50 | 4 | 4 | 4 | 4 | 0 | 1.000 | 1.000 | 1.000 | 1.00 | 1.25 | 0.00 | 1.0000 |
| 0.63 | 1 | 1 | 1 | 4 | 0 | 0.250 | 0.250 | 0.250 | 0.25 | 0.25 | 0.75 | 0.2500 |
| 0.16 | 0 | 0 | 0 | 4 | 0 | 0.000 | 0.000 | 0.000 | 0.00 | 0.00 | 1.75 | 0.0000 |

Actual drug concentration Table 9

| Drug name | Carrimycin | Dilution multiple | 4 | Drug name | 2019-nCoV | | | | Use | Treatment | IC50= | 0.50 |
|---|---|---|---|---|---|---|---|---|---|---|---|---|
| Concentration (ug/ml) | First row | Second row | Third row | Normal control | Virus control | | | | average | Accumulative | 1-accumulative | Cumulative inhibition rate% |
| 5.00 | 4 | 4 | 4 | 4 | 0 | 1.000 | 1.000 | 1.000 | 1.00 | 2.25 | 0.00 | 1.0000 |
| 1.25 | 4 | 4 | 4 | 4 | 0 | 1.000 | 1.000 | 1.000 | 1.00 | 1.25 | 0.00 | 1.0000 |
| 0.31 | 1 | 1 | 1 | 4 | 0 | 0.250 | 0.250 | 0.250 | 0.25 | 0.25 | 0.75 | 0.2500 |
| 0.08 | 0 | 0 | 0 | 4 | 0 | 0.000 | 0.000 | 0.000 | 0.00 | 0.00 | 1.75 | 0.0000 |

**Other experiments:**

Carrimycin can obviously improve the pathological changes of inflammation and edema in important organs of the whole body, especially lung, liver, kidney, heart, brain, stomach, intestine, etc., and can resist bacteria and improve inflammation and edema in vivo in immunocompromised nude mice. Carrimycin can play the role in resisting bacteria and improving tissue damage by starting the immune mechanism in vivo. For non-infectious inflammation, it can be seen that inflammation focus is alleviated, pain is alleviated or disappears, edema disappears, etc.; it can inhibit PI3K/Akt/mTOR pathway, and can significantly reduce the levels of immune factors such as IL-1$\beta$ and IL-4 in animals. It has the potential to inhibit M2 macrophages and improve the function of M1 macrophages. It has a strong function of inducing M2 macrophages to transform into M1 macrophages, while M2 macrophages promote inflammation.

See Fig. 17, the anti-inflammatory and immunomodulatory effects of carrimycin and isovalerylspiramycin I, isovalerylspiramycin II and isovalerylspiramycin III inhibit the PI3K/Akt/mTOR pathway, thus reducing IL-6 and IL-8 levels. The expression of mTOR and p-mTOR can be inhibited by treating A549 cells with carrimycin for 48 h, and the expression of P70S6K1 (ribosomal protein S6 kinase) and p-P70S6K1 downstream of mTOR can be inhibited by carrimycin. Studies have shown that the IL-6 and IL-8 levels will obviously decrease after mTOR is inhibited.

Novel coronavirus (2019-nCoV), like other coronaviruses, can stimulate the innate immune system of patients, causing a large number of cytokines to be released in the body, causing cytokine storm and acute inflammatory reaction. This will lead to more fragile blood vessels in the whole body and cause acute respiratory distress syndrome and multiple organ failure. Carrimycin of the present disclosure has an antiviral effect, and can be used in an early stage to reduce the effect of viruses on the immune system. For serious patients, by controlling cytokine storm and acute inflammatory reaction, as shown in Fig. 18, carrimycin can obviously reduce the damage to the lung and other organs.

Fig. 18 is a schematic diagram of the repair of lung injury by carrimycin. Within the range of clinical treatment dose (200-2000 mg/ person/day), the administration of carrimycin for 6 days can improve the tissue inflammatory reaction and tissue cell damage caused by infection in mice. HE pathological staining showed that carrimycin was more effective in improving inflammation and injury of the body tissues. It shows that carrimycin can improve the immune system function, repair tissue damage and promote rehabilitation.

Carrimycin has the characteristics of high safety, wide tissue distribution, high tissue concentration, long half-life and obvious antipyretic effect, also has the functions of anti-inflammatory, immune regulation, anti-edema, etc. It has a good therapeutic effect on drug-resistant Klebsiella pneumoniae and Acinetobacter baumannii, and has a potential clinical application value in the battle against coronavirus infection. Combining with the above results of molecular simulation, carrimycin can be considered to have a good clinical value in coronavirus infection, inflammatory reaction and secondary infection.

Clinical trials have been conducted in multiple hospitals through ethics to prove the effect of inhibiting 2019-nCoV.

As a major project of the Ministry of Science and Technology, a randomized, open, positive drug-controlled and multicenter clinical study was conducted on the curative effect and safety of oral administration of carrimycin in patients with COVID-19 (2019-nCoV), to provide important scientific basis for clinical effective and safe treatment.

Several designated hospitals in Beijing, Hubei, Liaoning and Heilongjiang in China for the treatment of 2019-nCoV participated in clinical research. The subjects, aged 18-75 years, met the diagnostic criteria of COVID-19 (the fifth edition). Patients have to meet any of the following requirements: (1) fever occurs again, or clinical symptoms worsen; (2) a nucleic acid test of throat swab turns from negative to positive; (3) clinical symptoms do not improve or nucleic acid continues to be positive; (5) chest CT shows lung inflammation or fibrosis progress. SOFA score: 1-13.

Treatment method

Mild type: carrimycin tablets, 0.4g each time, once a day, taken orally after meals, for 7 consecutive days, followed up for 30 days for observation after treatment.

Common type: carrimycin tablets, 0.4g each time, once a day, taken orally after meals, for 10 days, followed up for 30 days for observation after treatment.

Severe type and serious type: carrimycin tablets, 0.4g each time, once a day, taken orally after meals, for 14 consecutive days; for those who have difficulty in oral administration, the tablets were administrated through a nasal feeding tube. After treatment, the patients were followed up for 30 days for observation.

Main curative effect indicators: 1, fever clearance time (day); 2, lung inflammation resolution time (HRCT) (day); 3, negative conversion rate (%) of novel coronavirus in throat swabs on the 3rd and 7th days after treatment.

Secondary curative effect indicators: 1, description of clinical symptoms and adverse drug reactions; 2, changes of the peripheral white blood cell count relative to a baseline on the 1st, 3rd, 5th and 7th-10th day after medication; 3, changes of PCT and C-reactive protein relative to a baseline on the 1st, 3rd, 5th and 7th-10th days after medication; 4, changes of chest imaging characteristics relative to a baseline on the 7th-10th and 14th days after medication; 5, hospital stay and mortality.

General situation of enrolled cases

There were 47 cases of patients with "re-positive" of COVID-19 or treated patients, including 11 cases of mild type, 27 cases of common type, 3 cases of severe type and 6 cases of serious type. After prophase treatment, 40 cases were still positive for viral nucleic acid and 7 cases were negative for nucleic acid.
The main results were as follows:

(I) Main curative effect evaluation

1. Among 40 patients with positive viral nucleic acid, 16 patients turned negative in 3 days, 13 patients turned negative in 7 days, and 1 patient turned negative in 15 days. After treatment, 10 patients turned negative within 15 days.
2. There were 19 patients (7 cases with nucleic acid negative and 12 cases with nucleic acid positive) with pulmonary inflammation at the time of enrollment, and the significant improvement rate of pulmonary inflammation was 73.7% (14/19) in 7 days. Fig. 19 shows the improvement of pulmonary inflammation of one patient before and after treatment with carrimycin, which shows that the pulmonary inflammation improved significantly after 5 and 10 days of treatment with carrimycin; Fig. 20 shows the CT scannogram of another patient, wherein, (A) is the CT scannogram on the first day of illness, (B) is the CT scannogram on the fifth day of illness, (C) is the CT scannogram on the sixth day of illness (the beginning date of the treatment of carrimycin), and (D) is the CT scannogram on the eighth day of illness; (E) is the CT scannogram on the eleventh day of illness.
3. 5 patients (3 cases with nucleic acid negative and 2 cases with nucleic acid positive) had fever at the time of enrollment, the normalization rate of body temperature was 60% (3/5) in 3 days and 100% (5/5) in 7 days.

(II) Secondary curative effect evaluation

1. There were 11 patients with respiratory symptoms (2 cases with nucleic acid negative and 9 cases with nucleic acid positive) at the time of enrollment; the disappearance rate of respiratory symptoms was 36.4% (4/11) in 3 days and 100% (11/11) in 7 days.
2. There were 21 discharged patients, the cure and hospital discharge rate was 44.7% (21/47), and 9 patients were discharged within one week.
3. The enrolled patients did not get worse, and there were no deaths.

(III) Safety evaluation

No serious drug-related adverse reactions have been found, only one patient developed transient nausea on the 6th day after taking carrimycin, and a rash appeared on one patient, and the symptoms disappeared after the drug taking was stopped.
According to the above results, it can be preliminarily judged that for patients with "re-positive" of 2019-nCoV or treatment failure, carrimycin can quickly and effectively eliminate the novel coronavirus, significantly improve clinical symptoms and lung inflammation, improve the prognosis of patients suffering the COVID-19 pneumonia, and shorten the hospitalization time. Carrimycin is convenient for clinical application. It is administered orally once a day without obvious adverse reactions.

**Specific case analysis**

**Case 1:**

A patient, female, aged 72. Because of dry cough, fatigue, no fever and contact history, she was isolated and observed in the designated hospital for treating the COVID-19. After that, the patient suffered from severe dry cough and shortness of breath. She took lopinavir/ritonavir tablets, 400mg/100mg each time, twice a day, and was treated with oxygen inhalation for symptomatic treatment. Her symptoms did not relieve and she was admitted to a designated hospital in Beijing.
Laboratory examination: RT-PCR of throat swab showed that novel coronavirus was positive, oxygen saturation was 83% in oxygen inhalation state, oxygen partial pressure of blood gas analysis was 64mmHg, blood routine examination was normal, liver and kidney functions were normal, blood pressure was stable usually as the patient took antihypertensive drugs all the time due to the past history of high blood pressure. After admission, oxygen was inhaled through a nasal catheter and 0.4g of carrimycin was taken orally once a day. At the same time, nifedipine sustained-release tablets (30mg, once a day) and valsartan capsules (80mg, once a day), which are antihypertensive drugs, were

used. On the second day after admission, the patient's general condition improved, cough and dyspnea improved obviously, and oxygen saturation increased to 98%; oxygen partial pressure in blood gas analysis increased to 130mmHg, and nucleic acid test in throat swabs was negative on the 3rd and 6th day (February 25 and 27) after treatment with carrimycin. The CT (Fig. 21) showed that on the 6th day (the day before taking carrimycin), bilateral lung texture increased, irregular frosted glass-like lesions were found in the lower right lung field, and patchy shadows were scattered on the left side (arrow A). On the 9th day of the course (3 days after taking carrimycin), the bilateral lung texture was clear, and the irregular frosted glass-like lesions in the lower right lung field were obviously absorbed (indicated by arrow B). On the 12th day of the course (5 days after taking carrimycin), the lesions in the right lung were obviously absorbed (indicated by arrow C) and a small amount of fibrosis was formed. No adverse reactions were reported. After 6 days of hospitalization, she reached the discharge standard. Oral administration of carrimycin continued for 10 days after discharge, and there was no abnormality in the follow-up visit at present.

Experiences: 1, the disease progress of this elderly patient was found in time, although accompanied by hypertension and other diseases, the prognosis was good after timely treatment; 2, the treatment for this patient is very simple, and except for carrimycin and common antihypertensive drugs, no other drugs, including infusion, were used; 3, the patient has good compliance.

**Case 2:**

A patient, female, aged 49, has been taking anti-AIDS drugs due to being infected with HIV for 8 years. She has hypertension for 5 years with irregular treatment. The maximum admission temperature was 38DEG C, and auxiliary examination results and laboratory examination results were as below:

1. Blood routine: routine examination;
2. Influenza A virus antigen: negative;
3. Novel coronavirus nucleic acid test: positive;
4. Lung CT: right pneumonia lesion, viral pneumonia being not excluded, with localized thickening of bilateral pleura.

Diagnosis:

1. pneumonia infected by novel coronaviruses (common type);
2. AIDS;
3. Hypertension.

Main treatment conditions: the patient was treated with moxifloxacin for 3 days (400mg QD), interferon atomization for 16 days (5 million BID); ribavirin was taken orally for 10 days (150mg TID), followed by abidor for 10 days for antiviral treatment and Chinese herbal medicine treatment. During hospitalization, zidovudine and lamivudine tablets and efavirenz were regularly taken to treat HIV. Clinical symptoms improved: the patient had no cough, expectoration, chest tightness or shortness of breath and normal body temperature. Lung CT showed no obvious progress. After continuous treatment for 26 days, the novel coronavirus nucleic acid test for the sputum of the patient continued to be positive. 0.4g of carrimycin was orally taken, once a day, sputum and throat swabs were negative by novel coronavirus examination on the second and third days after administration. There was no discomfort during the treatment.

**Case 3:**

A patient, male, aged 41, was admitted to the hospital on February 9, 2020 due to "chest tightness and fatigue for 5 days". On February 10, the second novel coronavirus nucleic acid test result for the patient's throat swab was positive, so that the patient was diagnosed as suffering COVID-19. On February 15, 2020, the patient began to take carrimycin tablets (0.4g, qd), which is still used today (February 23). On February 16, 2020 (the first day after trial of the carrimycin tablets), blood routine reexamination showed that lymphocyte count was normal, myocardial enzymes were normal, and no obvious myocardial injury was found. On February 17, 2020 (the second day after the trial of the carrimycin tablets), the patient was in stable condition, feeling chest tightness after exercise, breathing stably at rest, and breathing oxygen through nasal catheter. Compared with the previous chest radiograph (February 9, 2020), the chest CT results showed that the range of interstitial lesions in the upper and middle lobes of the right lung was smaller and the density was lighter than before. On February 19, the test result of nucleic acid was negative. On February 21, 2020 (the 7th day after the trial of carrimycin tablets), the patient was in stable condition. On February 22 (the 8th day after the trial of carrimycin tablets), the interstitial lesions in the right upper and middle lobes of the right lung in the chest CT were slightly

absorbed than before (February 17, 2020). On February 22, the second nucleic acid test result was negative. He was discharged from hospital on February 24. No obvious adverse drug reactions occurred during the treatment.

**Case 4:**

A patient, male, aged 57, was admitted to the hospital for treatment on February 9, 2020 because of "cough for one week", which worsened on February 15, 2020. At the same time, the test result of novel coronavirus nucleic acid was positive. On February 19, 2020, other drugs were stopped and carrimycin tablets started to be administered for 0.4g, qd. On February 20, 2020 (the second day after the trial of carrimycin tablets), the patient informed of no cough, no fever, palpitation and chest tightness improved obviously. On February 25, 2020, the nucleic acid test result of the novel coronavirus was negative; chest CT was reexamined: infectious lesions in both lungs were partially absorbed and improved than before. On February 27, 2020, the nucleic acid test result of the novel coronavirus was negative. No adverse drug reactions occurred during the treatment.

**Case 5:**

A patient, female, aged 67, was admitted to the hospital on February 3, 2020 due to fever, and was diagnosed as suffering "COVID-19 (serious type)". She was given non-invasive ventilator assisted ventilation (2.6-2.19), oxygen inhalation (2.19-2.25), and treatment with antiviral drugs. On February 24, reexamination of the bedside chest radiograph showed that the two lungs had patchy clouding opacity and had poor absorption than before. The lymphocyte count was $0.82\times10^9$/L, and the blood gas analysis showed that the oxygen partial pressure was 64mmHg. On February 24, 2020, the antiviral drugs were stopped and the carrimycin was administered for treatment. On 2.26, reexamination showed that the chest CT was better than before, and on 2.27, reexamination showed that lymphocyte count was $1.09\times10^9$/L, and blood gas analysis showed that oxygen partial pressure rose to normal.

**Case 6:**

A patient, female, aged 69, was admitted to the hospital on February 3, 2020 due to fever, chest tightness and asthma. She was diagnosed as suffering "COVID-19 (serious type)" and given comprehensive treatment. The patient's body temperature was normal and the nucleic acid reexamination was negative. On February 24, reexamination of the bedside chest radiograph showed that the lungs were infected, and the absorption was not good compared with the previous one. The clinical manifestations were no fever, continuous cough and dyspnea, and continuous nasal high-flow oxygen inhalation with an oxygen concentration of 80% and an oxygen flow of 45L/min was provided. Carrimycin was taken on February 24, 2020. There was no fever after enrollment. On February 25, continuous nasal high-flow oxygen inhalation with an oxygen concentration of 60% and an oxygen flow rate of 35L/min was provided. On February 26, the oxygen concentration was 60% and the oxygen flow rate was 30L/min. On February 27, the oxygen concentration was 50%, and oxygen flow rate was 30L/min. On March 1, re-examination of bedside chest radiographs showed that it was better than before. On March 2, reexamination showed that the chest CT was improved than before.

The following table lists examples of other clinical studies on the treatment of COVID-19 with carrimycin.

Table 10 Other clinical studies of carrimycin in the treatment of COVID-19

| Center name | Case No. | Clinical typing | Case features | Enroll -ing time | Symptoms | Lung CT manifestation | Viral nucleic acid |
|---|---|---|---|---|---|---|---|
| Certain hospital in Beijing | 1 | Common | Viral nucleic acid was still positive after 6 days of hormone treatment | 2.21 | No fever Cough disappeared | Pneumonia absorption discharged | 2.24 nucleic acid turned negative 2.27 |
| | 2 | Severe | Initial treatment | 2.22 | Re No fever Cough disappeared | - examination of chest CT showed that the lesion area was reduced and the density was reduced | 2.25 nucleic acid turned negative 2.27 discharged |

(continued)

| Center name | Case No. | Clinical typing | Case features | Enroll -ing time | Symptoms | Lung CT manifestation | Viral nucleic acid |
|---|---|---|---|---|---|---|---|
| Certain hospital in Hubei | 1 | Severe | After 10 days of antiviral treatment with interferon and ribavirin, the virus nucleic acid was still positive; | 215 | No fever Chest tightness improved | Lesions were more absorbed than before | 2.19 nucleic acid turned negative |
| | 2 | Severe | 2.5 nucleic acid positive, abidor, kaletra antiviral treatment, 2.13 nucleic acid negative; 2.15 nucleic acid positive, no fever before enrollment | 2.19 | Chest tightness improved No cough | Lesions were more absorbed than before | 2.24 discharged 2.25 nucleic acid turned negative 2.27 retested nucleic acid was negative |
| | 3 | Common | Nucleic acid was still positive after antiviral treatment with kaletra, oseltamivir, ceftriaxone and ribavirin, no fever or clinical symptoms before enrollment | 2.29 | No obvious change | To be examined | 3.1 nucleic acid turned negative |
| | 4 | Common | Kaletra, ribavirin and interferon were not effective in antiviral treatment, no fever before enrollment, nucleic acid positive, occasional dizziness, cough, no expectoration, and white blood cell count and lymphocyte count were still low | 2.29 | No obvious change | To be examined | 3.1 nucleic acid turned negative |
| | 5 | Serious | The patient still had fever and a little cough after antiviral treatment with ribavirin, interferon and abidor | 2.18 | No fever, cough improved | 2.29 Chest CT showed more absorption than before | 2.26 throat swab negative 2.29 throat swab and anal swab negative 3.1 throat swab and urine negative |

(continued)

| Center name | Case No. | Clinical typing | Case features | Enroll -ing time | Symptoms | Lung CT manifestation | Viral nucleic acid |
|---|---|---|---|---|---|---|---|
| Certain hospital in Liaoning | 1 | Common | Lopinavir/ ritonavir oral administration, interferon atomization, virus nucleic acid were still positive | 2.20 | No fever, cough improved | Improved | 2.22 nucleic acid turned negative 2.24 discharged |
| | 2 | Common | Abidol, interferon atomization, and viral nucleic acid were still positive | 2.28 | Fever, cough improved | Improved | Turned negative |
| Certain hospital in Henan | 1 | Common | 1.24 Nucleic acid positive, negative twice after treatment, but still low fever, cough, asthma discomfort, poor imaging absorption, enrollment on the 20th, normal body temperature before enrollment | 2.24 | 2.27 body temperature 37.4DEGC | 2.26 Reexamine imaging showed more absorption than before | 2.28 nucleic acid negative 2.29 nucleic acid negative (3.2 discharged) |
| | 2 | Serious | After treatment, the nucleic acid turned negative, but the patient was still short of breath and dry cough, and the imaging was worse than before | 2.24 | No fever | 3.2 Reexamine CT showed more absorption than before | 2.28 nucleic acid negative |
| | 3 | Common | The nucleic acid test was positive (2.3), and the nucleic acid test was negative twice after treatment (2.13, 2.14). The reexamination of chest CT showed multiple grid-like changes in both upper lungs. The patient still had intermittent fever and asthma, and the imaging was still severe. The body temperature was normal before enrollment on February 20. | 2.24 | No fever | 2.26 Reexamine imaging showed more absorption than before discharged | 2.28 nucleic acid negative 2.28 |

(continued)

| Center name | Case No. | Clinical typing | Case features | Enroll -ing time | Symptoms | Lung CT manifestation | Viral nucleic acid |
|---|---|---|---|---|---|---|---|
| | 4 | Serious | (2.6) The patient suffered from shortness of breath and discomfort, and was given non-invasive ventilator assisted ventilation. The patient's body temperature was normal, and the nucleic acid was negative twice, but the patient still had respiratory failure, and the imaging was still heavy. | 2.24 | No fever | 2.26 Reexamine imaging showed more absorption than before | 2.28 nucleic acid negative 2.28 discharged |
| | 5 | Common | On February 6, the throat test sample was positive for nucleic acid. During hospitalization, it was found that the patient had venous thrombosis in lower limbs and increased blood sugar. Two consecutive nucleic acid tests were negative (February 23 and February 24), and the imaging examination showed no absorption | 2.24 | No fever | Not reexamined yet | 2.28 nucleic acid negative 2.28 discharged |
| | 6 | Common | 2.3 nucleic acid was positive, 2.10 nucleic acid was uncertain, 2.12 nucleic acid was negative, 2.17 nucleic acid was positive, 2.19 and 2.20 nucleic acid were uncertain, and the patient still felt fatigue | 2.24 | No fever | Not reexamined yet | Negative (2.25 discharged ) |

(continued)

| Center name | Case No. | Clinical typing | Case features | Enroll -ing time | Symptoms | Lung CT manifestation | Viral nucleic acid |
|---|---|---|---|---|---|---|---|
| | 7 | Common | 2.4 nucleic acid was positive. After treatment, the nucleic acid was positive twice, and the third nucleic acid reexamination showed: uncertain | 2.24 | No fever | Not reexamined yet withdrawn) | 2.25 positive, 2.26 positive nucleic acid negative, 2.28 negative (allergy, |
| | 8 | Common | Initial treatment | 2.24 | No fever | Not reexamined yet | 2.24, 2.25 nucleic acid negative (2.26 discharged ) |
| | 9 | Common | 2.12 nucleic acid test was positive, 2.19, 2.20 reexamine nucleic acid was positive. | 2.25 | No fever | Not reexamined yet | 2.25 nucleic acid negative, 2.26 ORF-lab gene negative, N gene negative, uncertain result; 2.27 ORF-lab gene negative, N gene positive, uncertain result, 2.28, 2.29 nucleic acid negative |
| | 10 | Common | 2.14 nucleic acid test was positive, 2.20 nucleic acid was positive, 2.21 nucleic acid was uncertain, 2.23 negative, 2.24 uncertain, 2.26 positive, 2.27 ORF-lab gene was negative, N gene was positive, and the result was uncertain | 2.27 | No fever | Not reexamined yet | 2.29 nucleic acid negative |

(continued)

| Center name | Case No. | Clinical typing | Case features | Enroll -ing time | Symptoms | Lung CT manifestation | Viral nucleic acid |
|---|---|---|---|---|---|---|---|
| | 11 | Common | 1.30 nucleic acid was positive, 2.4 and 2.5 nucleic acid was negative, 2.19 positive, 2.22 and 2.23 nucleic acid was negative, and 2.24 nucleic acid in feces was positive | 2.27 | No fever | Not reexamined yet | 2.29 nucleic acid negative |
| | 12 | Common | 1.29 nucleic acid positive, 2.7 and 2.8 nucleic acid negative, 2.21 nucleic acid positive, 2.22 and 2.23 nucleic acid negative and 2.24 nucleic acid in feces positive | 2.27 | No fever | Not reexamined yet | 2.29 nucleic acid negative |

In addition, carrimycin is obvious in curative effect of treating COVID-19 pneumonia in the clinical trials. Thirty-two patients (one new serious type patient for trial use) in a city hospital and its affiliated county hospitals in Hubei Province were treated with carrimycin 400mg, qd on the basis of antiviral medicines recommended by Guidelines for Diagnosis and Treatment of COVID-19 (the 5th Editi*on*). On the 3rd to 5th day after medication, the body temperature of 3 patients returned to normal, 3 patients had repeated fever, and 26 patients had no fever symptoms before using carrimycin. In 8 patients who failed in antiviral treatment, the negative conversion rate of 2019nCOVRNA in throat swabs was 100% on the 3rd to 10th day after medication.

## Claims

1. Application of an antibiotic, especially a macrolide antibiotic compound, in manufacturing medicine for treating coronavirus infection disease.

2. The application according to claim 1, wherein, the antibiotic is an acylated spiramycin compound or a composition of the acylated spiramycin compound;
preferably, the application of an isovalerylspiramycin compound or a composition of the isovalerylspiramycin compound in manufacturing medicine for treating coronavirus infection disease.

3. The application according to claim 1, wherein, the antibiotic is selected from one of isovalerylspiramycin I, isovalerylspiramycin II and isovalerylspiramycin III; or a combination of at least two of isovalerylspiramycin I, isovalerylspiramycin II and isovalerylspiramycin III; or carrimycin; structures of the isovalerylspiramycin I, isovalerylspiramycin II and isovalerylspiramycin III are as the following formula:

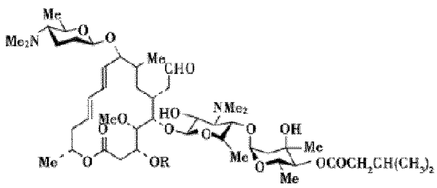

R= H, spiramycin I or 4"-isovalerylspiramycin I ;
R= $CH_3CO$, spiramycin II or 4"-isovalerylspiramycin II ;
R= $CH_3CH_2CO$, spiramycin III or 4"-isovalerylspiramycin III

.

4. The application according to claim 1, wherein, coronavirus in the coronavirus infection disease comprises α and β coronaviruses,

Preferably, HCoV-229E, HCoV-OC43, SARS-CoV, HCoV-NL63, HCoV-HKU1, MERS-CoV and/or 2019-nCoV, and more preferably SARS-CoV, MERS-CoV and/or 2019-nCoV.

5. The application according to any one of claims 1 to 4, wherein, the coronavirus infection disease comprises respiratory tract disease, digestive tract disease and nervous system disease, including but not limited to cold, fever, frontal sinusitis, otitis media, pharyngitis, chronic bronchitis, pneumonia, pleural effusion, various respiratory syndromes, acute gastroenteritis, cardiopulmonary diseases, hypoimmunity, repeated infection, lung injury, organ failure and other diseases,
especially various diseases caused by SARS, MERS and 2019-nCoV.

6. The application according to any one of claims 1 to 4, wherein, the acylated spiramycin compound or the composition of the acylated spiramycin compound binds to a main protease Mpro inhibition site of coronavirus.

7. The application according to any one of claims 1 to 4, wherein, the acylated spiramycin compound or the composition of the acylated spiramycin compound binds to coronavirus S protein and a receptor ACE2 protein of a host cell of the coronavirus.

8. The application according to any one of claims 1 to 5, wherein, the coronavirus infection disease comprises complications caused by the coronavirus infection disease.

9. The application according to any one of claims from 1 to 8, comprising a first active pharmaceutical ingredient selected from one of acylated spiramycin compounds, or a combination of acylated spiramycin compounds, or carrimycin, and a second active pharmaceutical ingredient;
the second active pharmaceutical ingredient is selected from antiviral medicine and/or anti-AIDS medicine, and the first active pharmaceutical ingredient and the second active pharmaceutical ingredient are prepared separately or compounded into one preparation.

10. The application according to any one of claims from 1 to 9, comprising a first active pharmaceutical ingredient selected from one of acylated spiramycin compounds, or a combination of acylated spiramycin compounds, or carrimycin, and a second active pharmaceutical ingredient; the second active pharmaceutical ingredient is selected from at least one of a protease inhibitor, a fusion protein inhibitor, a nucleoside reverse transcriptase inhibitor and an immunosuppressant.

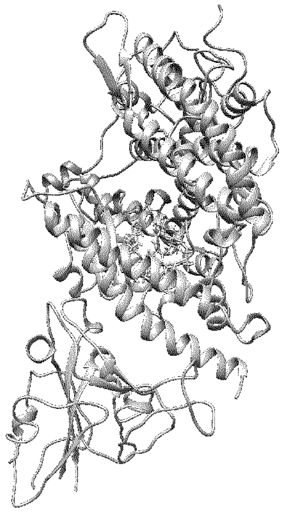

FullFitness: -3458.021 kcal/mol
deltaG: -10.978372 kcal/mol

ACE2 (*Homo sapiens*) and spike protein
PDB: 3SCJ
Resolution: 3.0 Å

Fig. 1

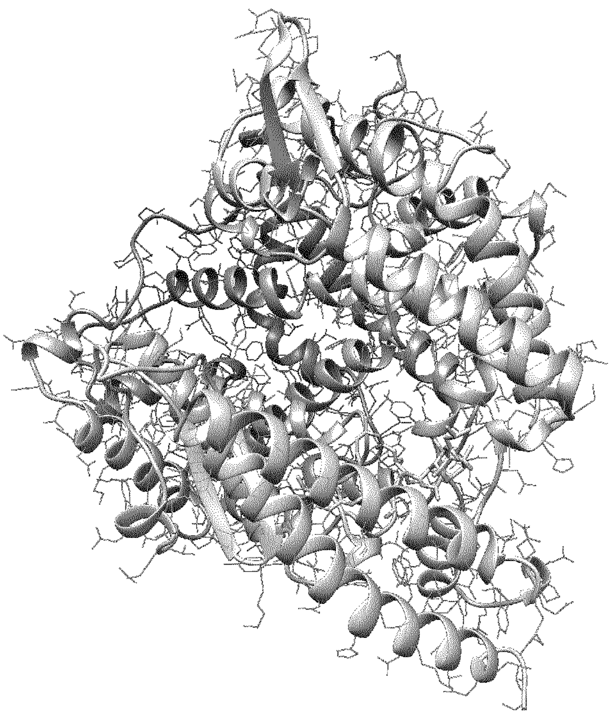

Energy: 93.9319 kcal/mol
SimpleFitness: 93.9319 kcal/mol
FullFitness: -2883.819 kcal/mol
InterFull: -115.704 kcal/mol
IntraFull: 213.905 kcal/mol
solvFull: -3386.81 kcal/mol
surfFull: 404.79 kcal/mol
extraFull: 0.0 kcal/mol
deltaGcompsolvpol: -3386.81 kcal/mol
deltaGcompsolvnonpol: 404.79 kcal/mol
deltaGprotsolvpol: -3404.87 kcal/mol
deltaGprotsolvnonpol: 407.132 kcal/mol
deltaGligsolvpol: -70.2345 kcal/mol
deltaGligsolvnonpol: 20.5553 kcal/mol
deltaGvdw: -115.704 kcal/mol
deltaGelec: 0.0 kcal/mol
deltaG: -11.698055 kcal/mol
Cluster: 23
ClusterRank: 0

ACE (*Homo sapiens*)
PDB: 1R42
Resolution: 2.2 Å

Compound: isovalerylspiramycin I (one of the main active ingredients of carrimycin)

Strong binding to human ACE2 protein

Fig. 2

3a

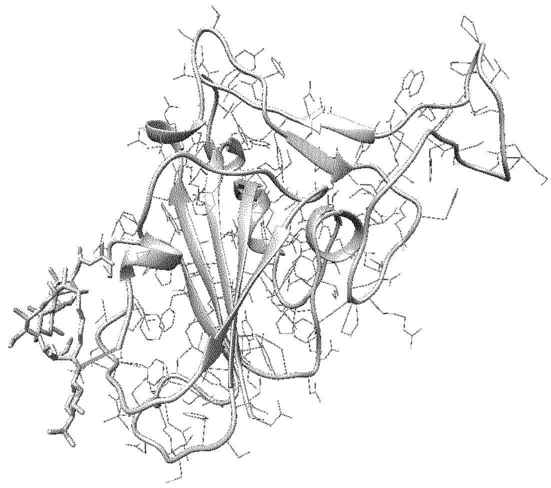

Energy: 121.091 kcal/mol
SimpleFitness: 121.091 kcal/mol
FullFitness: -683.793 kcal/mol
InterFull: -38.775 kcal/mol
IntraFull: 213.056 kcal/mol
solvFull: -1020.02 kcal/mol
surfFull: 161.946 kcal/mol
extraFull: 0.0 kcal/mol
deltaGcompsolvpol: -1020.02 kcal/mol
deltaGcompsolvnonpol: 161.946 kcal/mol
deltaGprotsolvpol: -974.296 kcal/mol
deltaGprotsolvnonpol: 154.031 kcal/mol
deltaGligsolvpol: -73.9795 kcal/mol
deltaGligsolvnonpol: 21.1041 kcal/mol
deltaGvdw: -38.775 kcal/mol
deltaGelec: 0.0 kcal/mol
deltaG: -7.176568 kcal/mol
Cluster: 2
ClusterRank: 6

SARS coronavirus spike protein
PDB: 3BGF
Resolution: 3 Å

Compound: isovalerylspiramycin I (one of the main active ingredients of carrimycin)
Weak binding to SARS spike protein

3b

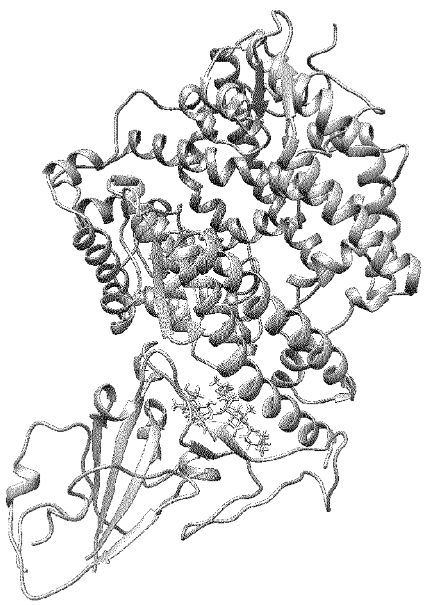

FullFitness: -3438.682 kcal/mol
deltaG: -8.078679 kcal/mol

ACE2 (*Homo sapiens*) and spike protein
PDB: 3SCJ
Resolution: 3.0 Å

Fig. 3

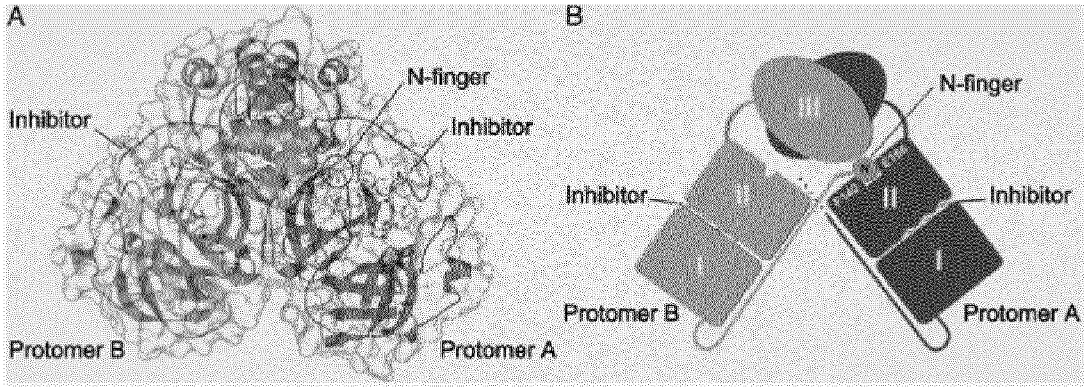
A
Inhibitor
N-finger
Inhibitor
Protomer B
Protomer A
B
N-finger
Inhibitor
Inhibitor
Protomer B
Protomer A

Fig. 4

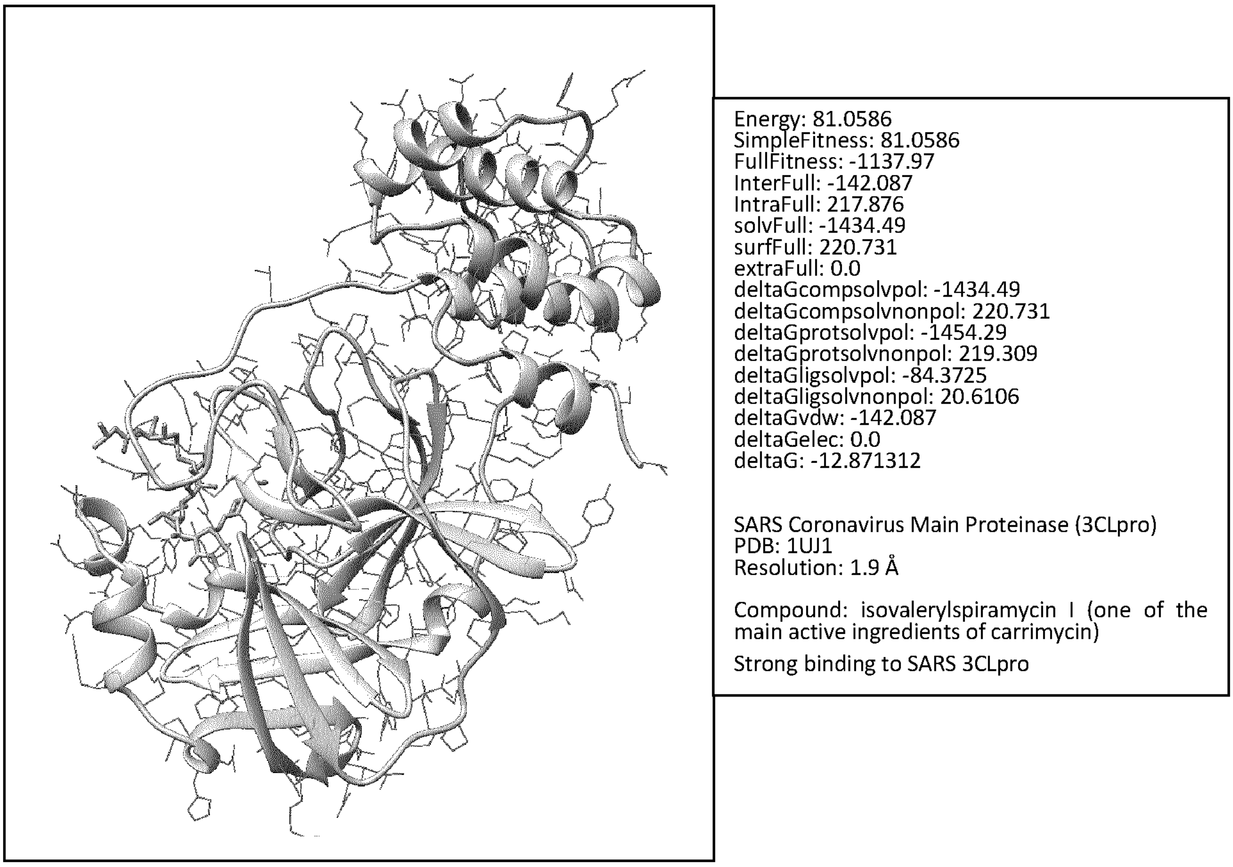
Energy: 81.0586
SimpleFitness: 81.0586
FullFitness: -1137.97
InterFull: -142.087
IntraFull: 217.876
solvFull: -1434.49
surfFull: 220.731
extraFull: 0.0
deltaGcompsolvpol: -1434.49
deltaGcompsolvnonpol: 220.731
deltaGprotsolvpol: -1454.29
deltaGprotsolvnonpol: 219.309
deltaGligsolvpol: -84.3725
deltaGligsolvnonpol: 20.6106
deltaGvdw: -142.087
deltaGelec: 0.0
deltaG: -12.871312
SARS Coronavirus Main Proteinase (3CLpro)
PDB: 1UJ1
Resolution: 1.9 Å
Compound: isovalerylspiramycin I (one of the main active ingredients of carrimycin)
Strong binding to SARS 3CLpro

Fig. 5

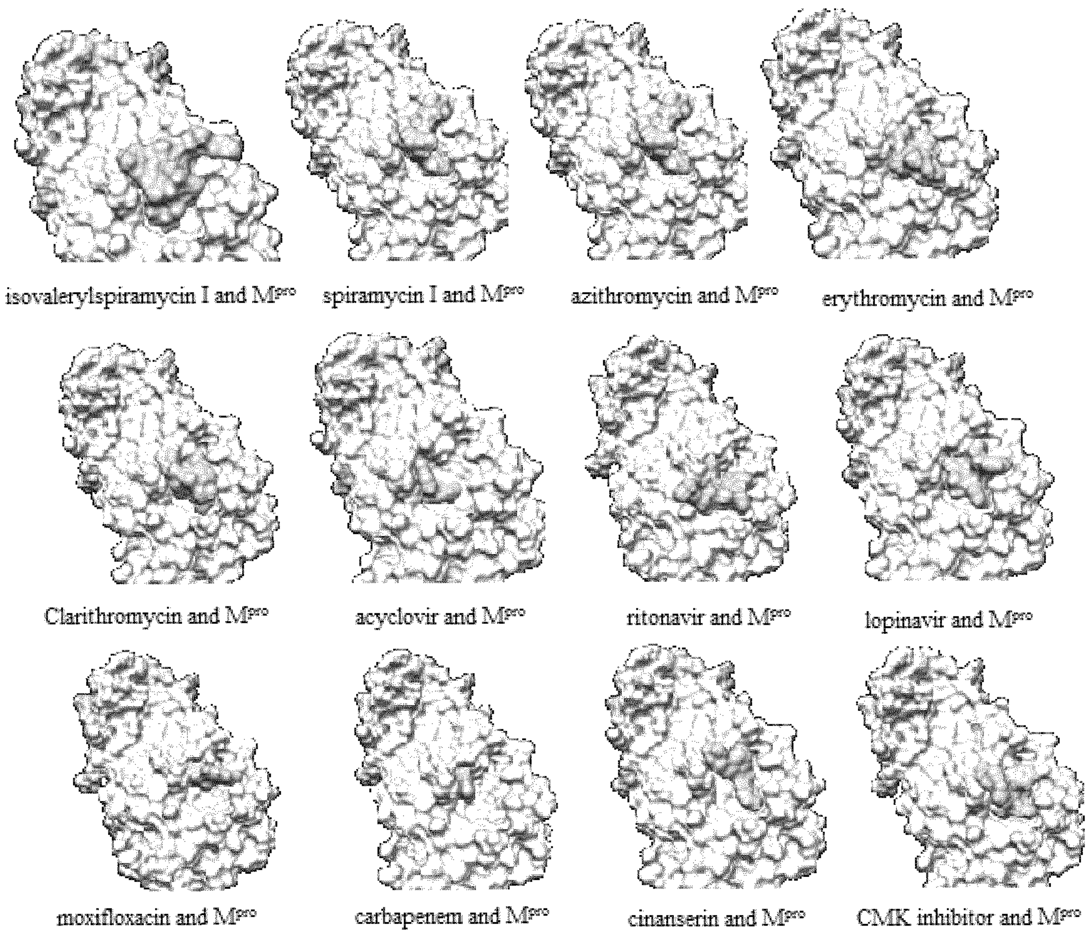


Fig. 6

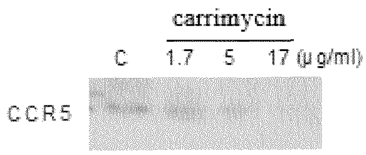


Fig. 7

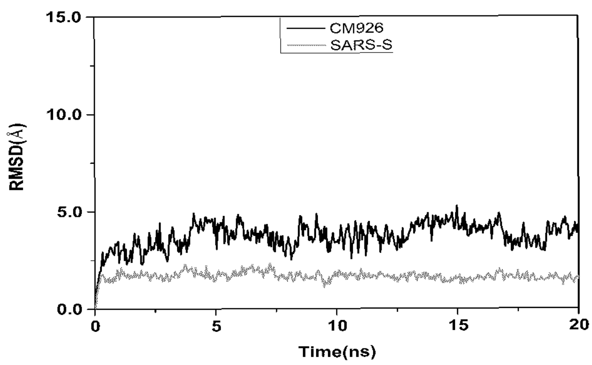


Fig. 8

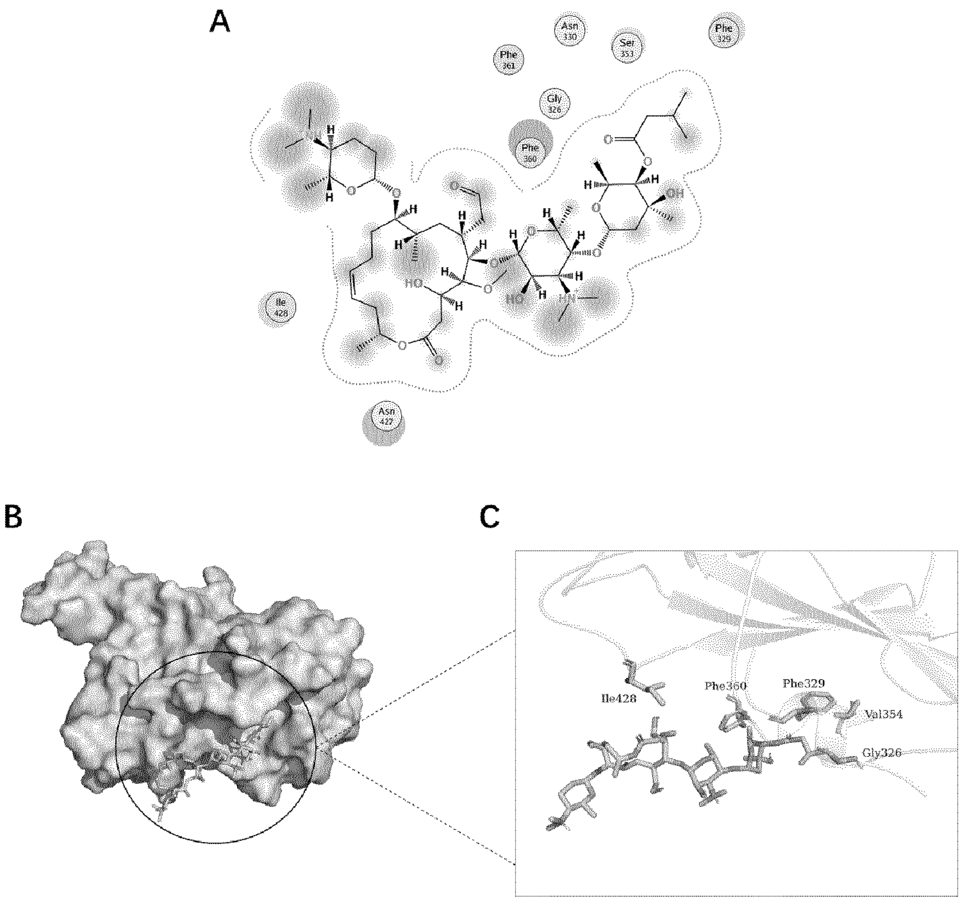
A
B
C
Ile428
Phe360
Phe329
Val354
Gly326

Fig. 9

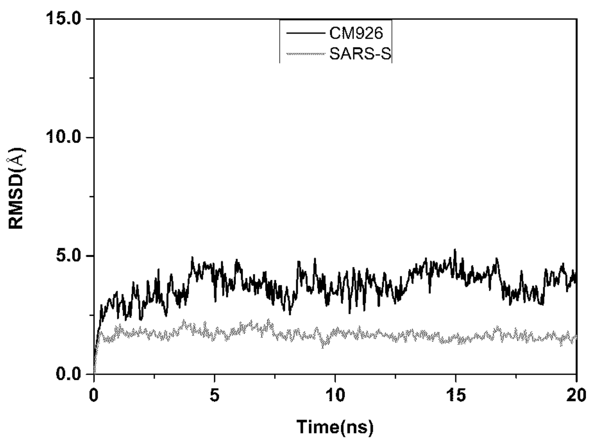
CM926
SARS-S
15.0
10.0
5.0
0.0
RMSD(Å)
0
5
10
15
20
Time(ns)

Fig. 10

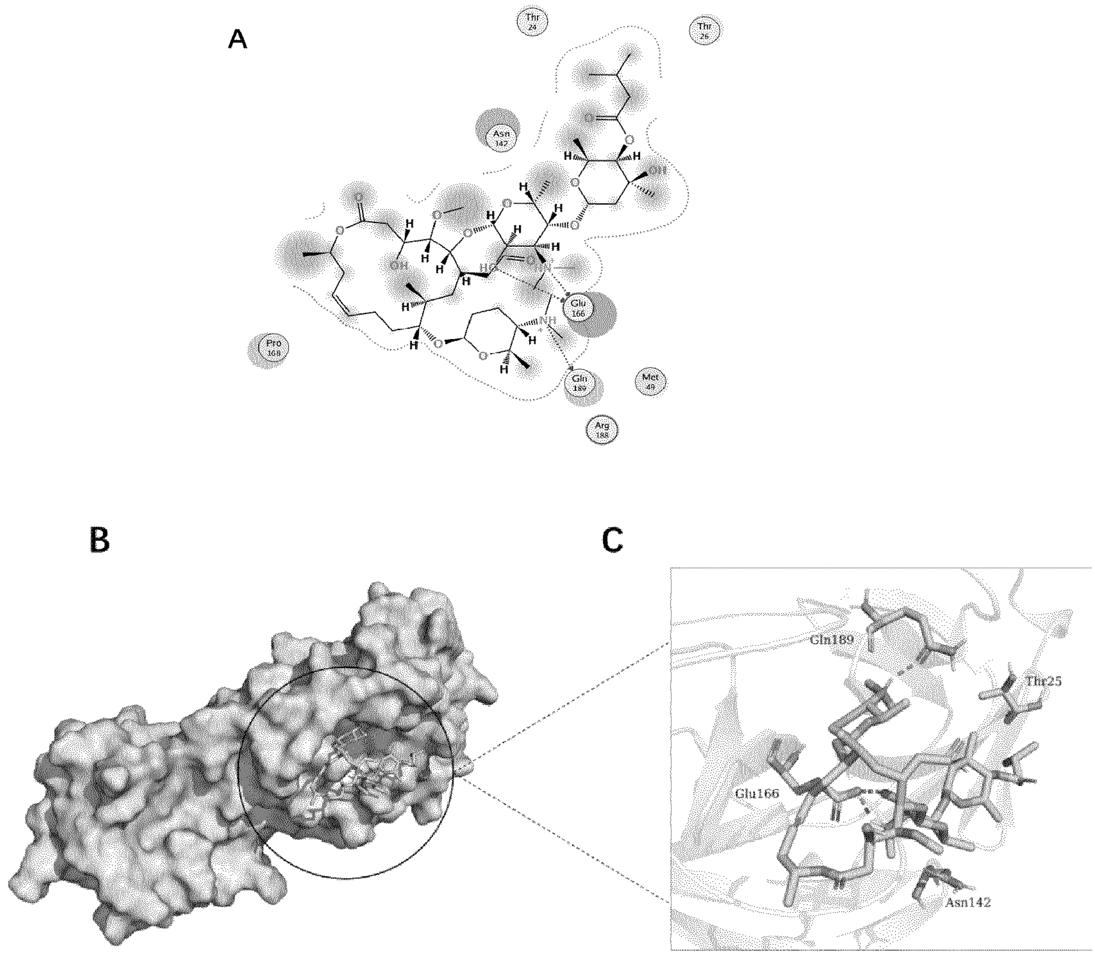
A
B
C
Gln189
Thr25
Glu166
Asn142

Fig. 11

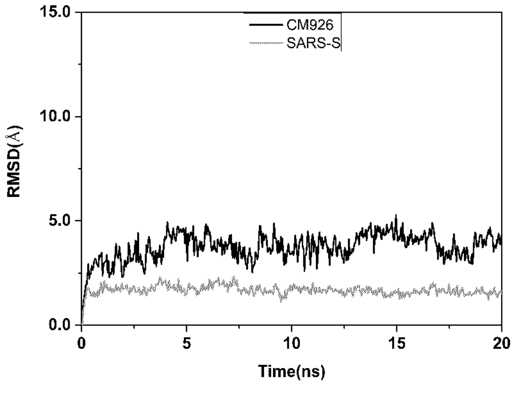
CM926
SARS-S
RMSD(Å)
Time(ns)
15.0
10.0
5.0
0.0
0
5
10
15
20

Fig. 12

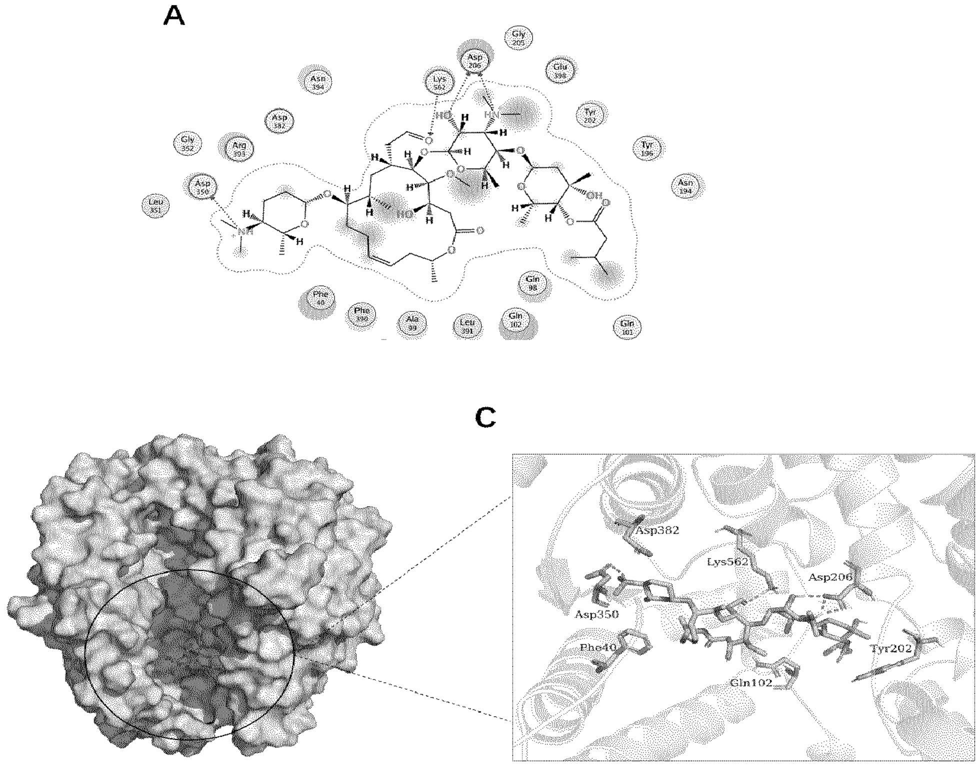
A
B
C
Asp382
Lys562
Asp206
Asp350
Phe40
Tyr202
Gln102

Fig. 13

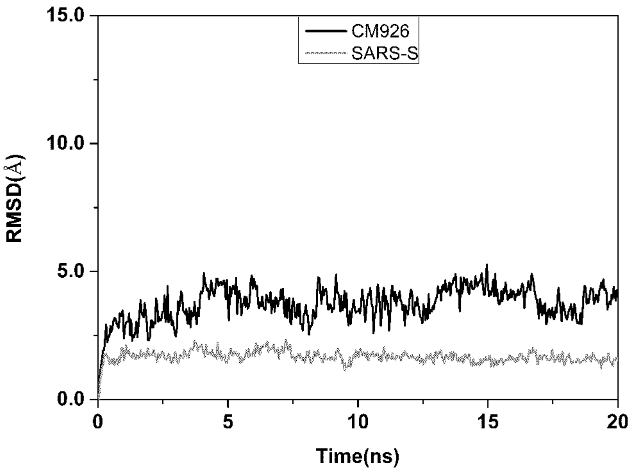
CM926
SARS-S
RMSD(Å)
Time(ns)
15.0
10.0
5.0
0.0
0
5
10
15
20

Fig. 14

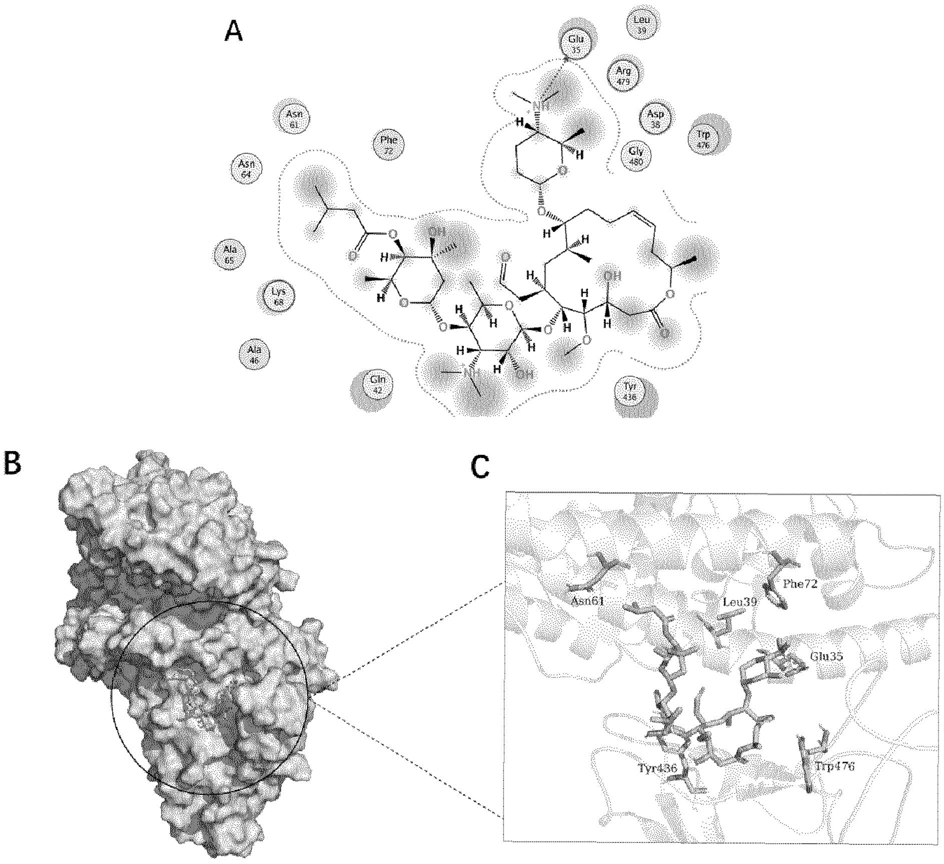
A
B
C
Asn61
Leu39
Phe72
Glu35
Tyr436
Trp476

Fig. 15

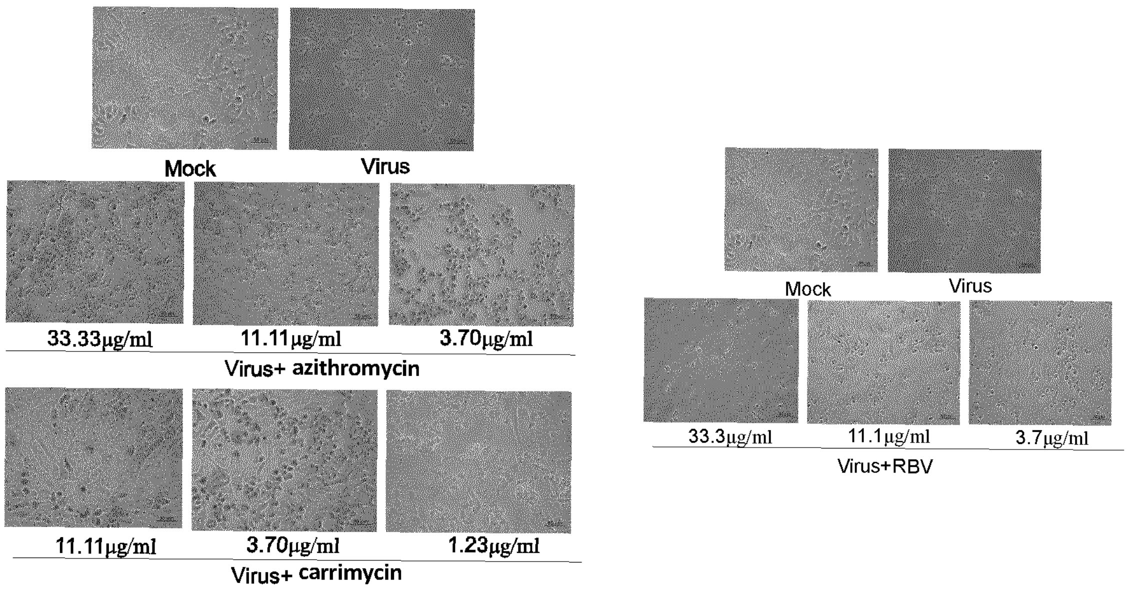
Mock
Virus
33.33μg/ml
11.11μg/ml
3.70μg/ml
Virus+ azithromycin
11.11μg/ml
3.70μg/ml
1.23μg/ml
Virus+ carrimycin
Mock
Virus
33.3μg/ml
11.1μg/ml
3.7μg/ml
Virus+RBV

Fig. 16

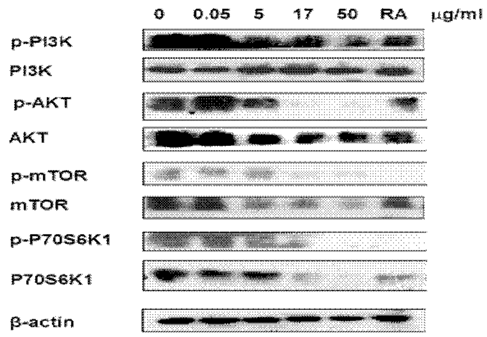
0
0.05
5
17
50
RA
μg/ml
p-PI3K
PI3K
p-AKT
AKT
p-mTOR
mTOR
p-P70S6K1
P70S6K1
β-actin

Fig. 17

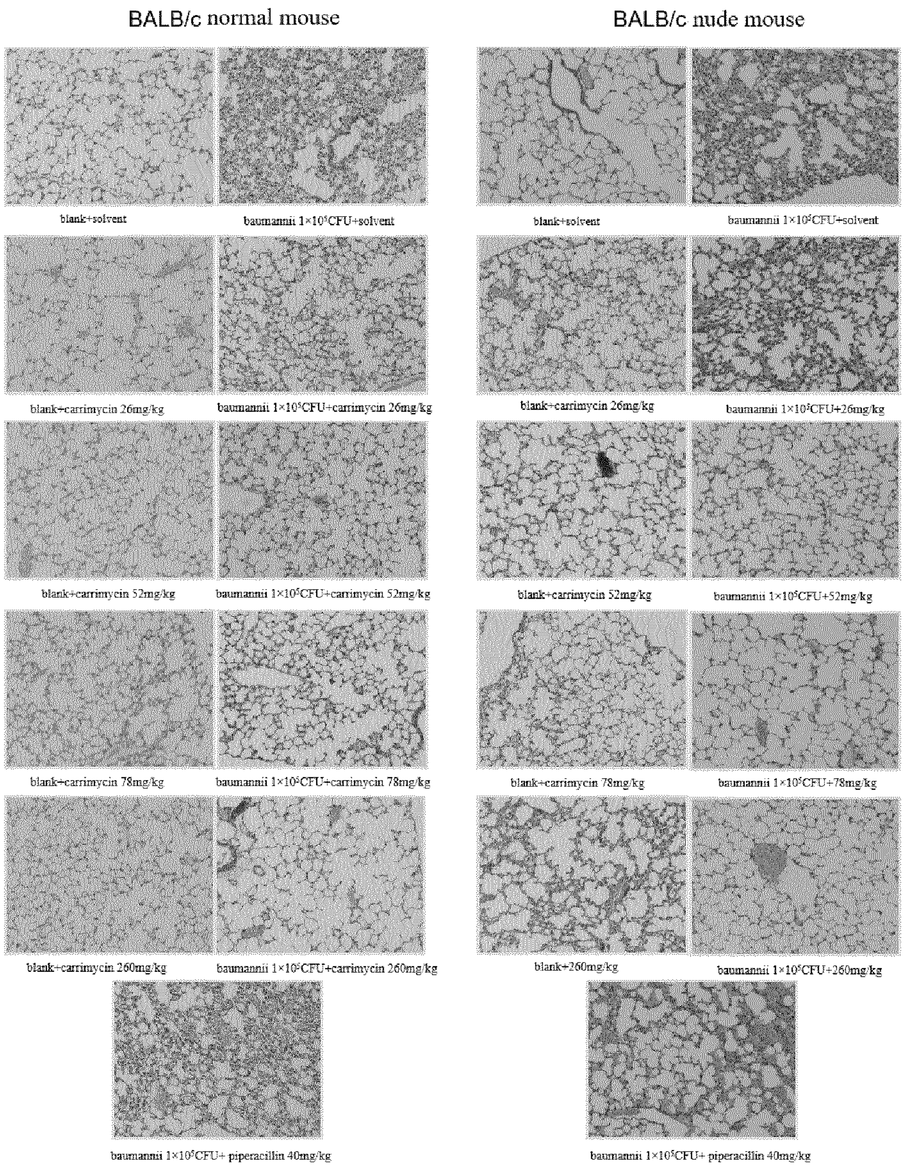
BALB/c normal mouse
BALB/c nude mouse
blank+solvent
baumannii 1×10$^5$CFU+solvent
blank+solvent
baumannii 1×10$^5$CFU+solvent
blank+carrimycin 26mg/kg
baumannii 1×10$^5$CFU+carrimycin 26mg/kg
blank+carrimycin 26mg/kg
baumannii 1×10$^5$CFU+26mg/kg
blank+carrimycin 52mg/kg
baumannii 1×10$^5$CFU+carrimycin 52mg/kg
blank+carrimycin 52mg/kg
baumannii 1×10$^5$CFU+52mg/kg
blank+carrimycin 78mg/kg
baumannii 1×10$^5$CFU+carrimycin 78mg/kg
blank+carrimycin 78mg/kg
baumannii 1×10$^5$CFU+78mg/kg
blank+carrimycin 260mg/kg
baumannii 1×10$^5$CFU+carrimycin 260mg/kg
blank+260mg/kg
baumannii 1×10$^5$CFU+260mg/kg
baumannii 1×10$^5$CFU+ piperacillin 40mg/kg
baumannii 1×10$^5$CFU+ piperacillin 40mg/kg

Fig. 18

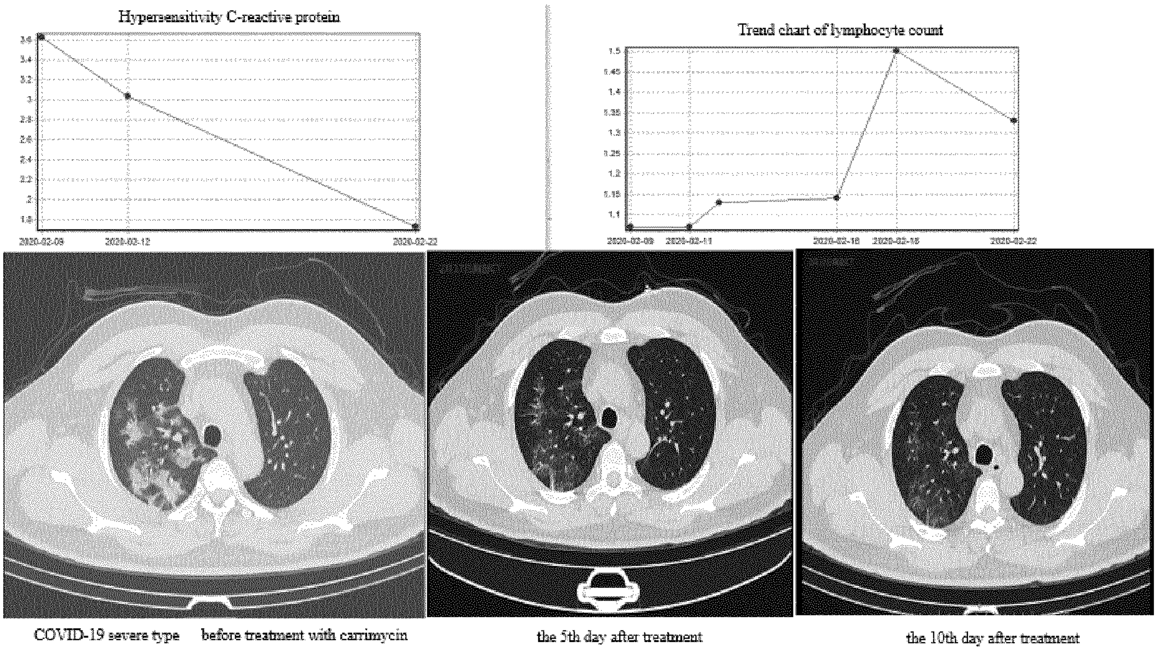
Hypersensitivity C-reactive protein
Trend chart of lymphocyte count
COVID-19 severe type before treatment with carrimycin
the 5th day after treatment
the 10th day after treatment

Fig. 19

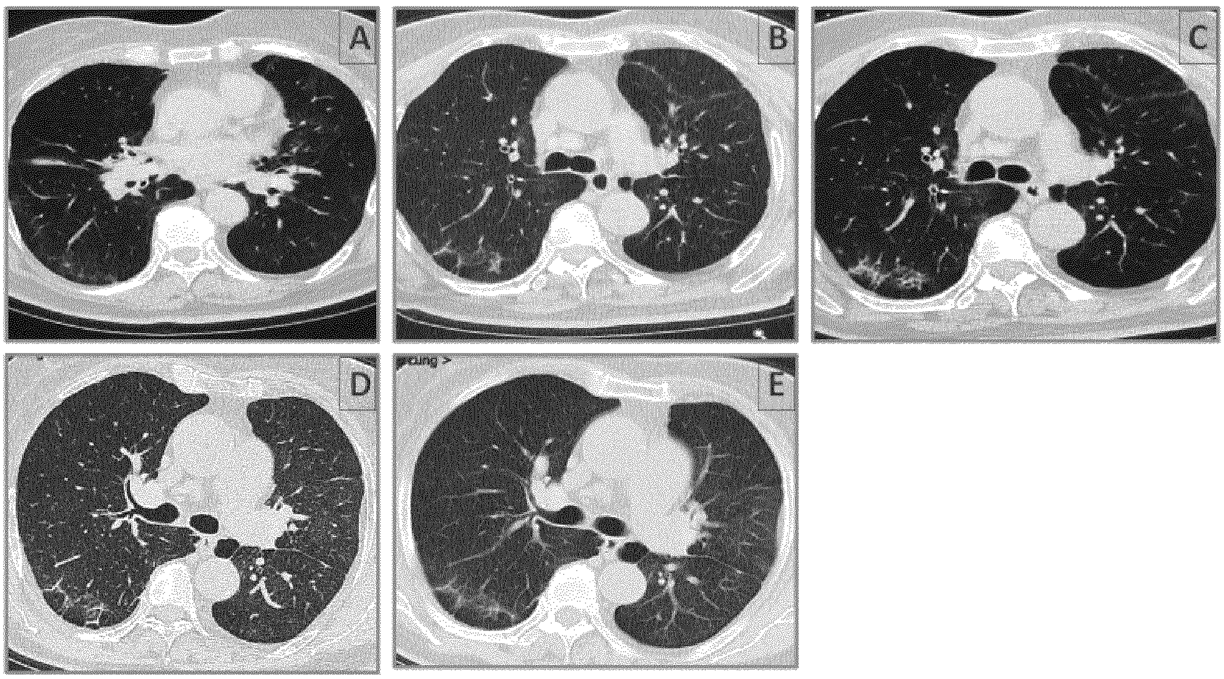
A
B
C
D
E

Fig. 20

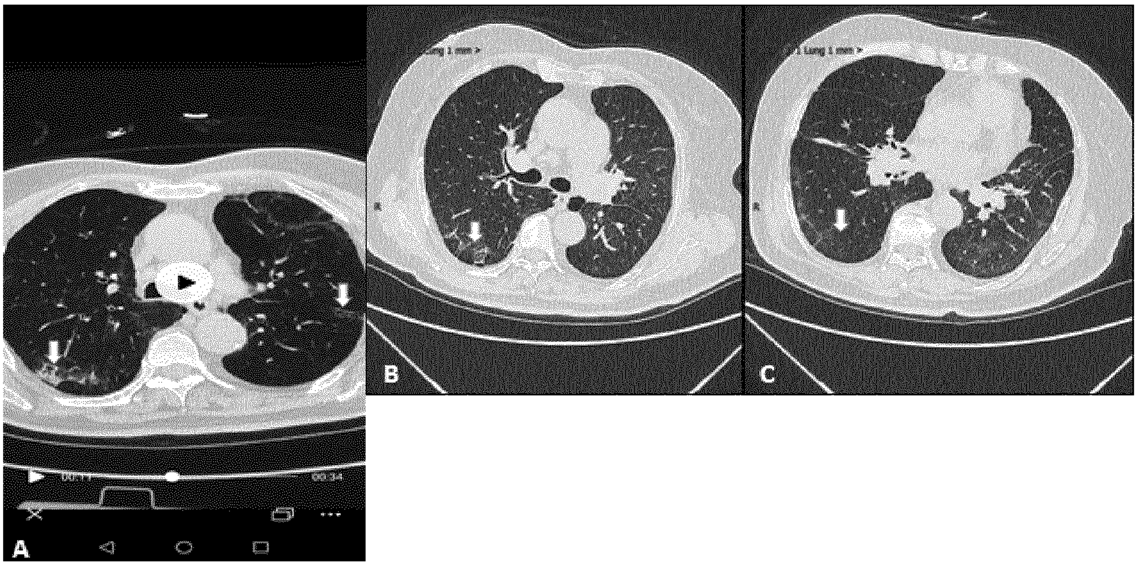


Fig. 21

**INTERNATIONAL SEARCH REPORT**

International application No.
**PCT/CN2020/081204**

**A. CLASSIFICATION OF SUBJECT MATTER**

A61K 31/7048(2006.01)i；A61K 45/06(2006.01)i；A61P 31/14(2006.01)i；A61P 31/18(2006.01)i

According to International Patent Classification (IPC) or to both national classification and IPC

**B. FIELDS SEARCHED**

Minimum documentation searched (classification system followed by classification symbols)

A61K，A61P

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, 中国药物专利数据库, CNKI, 百度学术搜索, 超星读秀学术(CN), 万方(CN), Medline, ISI-WEB OF SCIENCE, EMBASE, CHEMICAL ABSTRACTS: 冠状, 可利霉素, 螺旋霉素, 抗生素, 病毒, Macrolide?, 冠状, Corona virus?, 冠状病毒, Anti?biotic?, Corona?virus?, colimycin, spiramycin

**C. DOCUMENTS CONSIDERED TO BE RELEVANT**

| Category* | Citation of document, with indication, where appropriate, of the relevant passages | Relevant to claim No. |
|---|---|---|
| X | CN 1921865 A (BOEHRINGER INGELHEIM VETMEDICA GMBH) 28 February 2007 (2007-02-28) see claims 1, 6, 10 | 1, 4, 5 |
| X | CN 101511374 A (ECO ANIMAL HEALTH LTD.; CAMBRIDGE UNIV. TECHNICAL SERVICES LTD.) 19 August 2009 (2009-08-19) see claims 1, 7 | 1, 4 |
| A | CN 1921865 A (BOEHRINGER INGELHEIM VETMEDICA GMBH) 28 February 2007 (2007-02-28) see claims 1, 6, 10 | 2-3, 6-10 |

☐ Further documents are listed in the continuation of Box C. ☑ See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **23 October 2020** | **30 October 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/081204**

| Patent document cited in search report | Publication date (day/month/year) | Patent family member(s) | Publication date (day/month/year) |
|---|---|---|---|
| CN 1921865 A | 28 February 2007 | RU 2006133829 A | 27 March 2008 |
| | | CN 1921865 B | 16 June 2010 |
| | | RU 2360680 C2 | 10 July 2009 |
| | | WO 2005079806 A1 | 01 September 2005 |
| | | US 10548901 B2 | 04 February 2020 |
| | | ES 2394038 T3 | 16 January 2013 |
| | | EP 1720554 A1 | 15 November 2006 |
| | | CA 2554040 C | 05 August 2014 |
| | | US 2020121692 A1 | 23 April 2020 |
| | | TW 200533362 A | 16 October 2005 |
| | | CA 2554040 A1 | 01 September 2005 |
| | | US 2018185380 A1 | 05 July 2018 |
| | | KR 20070033960 A | 27 March 2007 |
| | | AU 2005215122 B2 | 11 August 2011 |
| | | KR 101233359 B1 | 13 February 2013 |
| | | MX PA06009575 A | 15 March 2007 |
| | | EP 1568369 A1 | 31 August 2005 |
| | | US 2014179639 A1 | 26 June 2014 |
| | | DK 1720554 T3 | 15 October 2012 |
| | | EP 1720554 B1 | 18 July 2012 |
| | | US 2005187213 A1 | 25 August 2005 |
| | | UA 92584 C2 | 25 November 2010 |
| | | BR PI0507966 A | 17 July 2007 |
| | | US 2008280840 A1 | 13 November 2008 |
| | | JP 5009147 B2 | 22 August 2012 |
| | | AU 2005215122 A1 | 01 September 2005 |
| | | SG 150531 A1 | 30 March 2009 |
| | | JP 2007523205 A | 16 August 2007 |
| CN 101511374 A | 19 August 2009 | CN 101511374 B | 04 July 2012 |
| | | GB 0613952 D0 | 23 August 2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Guidelines for Diagnosis and Treatment of COVID-19